# EUROPEAN PATENT SPECIFICATION

(11) **EP 2 534 487 B1**
(45) Date of publication and mention of the grant of the patent: **04.11.2015**
(21) Application number: 11711144.3
(22) Date of filing: 13.02.2011
(51) Int. Cl.: G01N 33/564

(54) **DIAGNOSIS OF SYSTEMIC LUPUS ERYTHEMATOSUS (SLE)**
VERFAHREN ZUR DIAGNOSE VON SYSTEMISCHEM LUPUS ERYTHEMASTOSUS (SLE)
MÉTHODE DE DIAGNOSTIC DU LUPUS ÉRYTHÉMATEUX SYSTÉMIQUE (LES)

(30) Priority: 12.02.2010 US 303691 P
(43) Date of publication of application: 19.12.2012
(73) Proprietor: Yeda Research and Development Co. Ltd., 76100 Rehovot (IL); Tel HaShomer Medical Research Infrastructure and Services Ltd., 52 621 Ramat Gan (IL)
(72) Inventor: COHEN, Irun R., 76354 Rehovot (IL); DOMANY, Eytan, 76100 Rehovot (IL); SHENTAL, Noam, 76100 Rehovot (IL); FATTAL, Ittai, 76100 Rehovot (IL)
(74) Representative: Becker Kurig Straus
(86) International application number: PCT/IL2011/000153
(87) International publication number: WO 2011/099012

(56) References cited:
- Q-Z LI ET AL: "Protein array autoantibody profiles for insights into systemic lupus erythematosus and incomplete lupus syndromes", CLINICAL AND EXPERIMENTAL IMMUNOLOGY, WILEY-BLACKWELL PUBLISHING LTD, GB, vol. 147, no. 1, 1 January 2007 (2007-01-01), pages 60-70, XP002622508, ISSN: 0009-9104, DOI: DOI:10.1111/J.1365-2249.2006.03251.X [retrieved on 2006-11-27]
- ROBINSON W H ET AL: "Autoantigen microarrays for multiplex characterization of autoantibody responses", NATURE MEDICINE, NATURE PUBLISHING GROUP, NEW YORK, NY, US, vol. 8, no. 3, 1 March 2002 (2002-03-01), pages 295-301, XP002244630, ISSN: 1078-8956, DOI: DOI:10.1038/NM0302-295
- HANLY JOHN G ET AL: "Measurement of autoantibodies using multiplex methodology in patients with systemic lupus erythematosus", JOURNAL OF IMMUNOLOGICAL METHODS, vol. 352, no. 1-2, January 2010 (2010-01), pages 147-152, XP002637354, ISSN: 0022-1759
- QUINTANA F J ET AL: "Antigen-chip technology for accessing global information about the state of the body", LUPUS, vol. 15, no. 7, 2006, pages 428-430, XP009148423, ISSN: 0961-2033
- FATTAL ITTAI ET AL: "An antibody profile of systemic lupus erythematosus detected by antigen microarray", IMMUNOLOGY, vol. 130, no. 3, July 2010 (2010-07), pages 337-343, XP002637355, ISSN: 0019-2805

## Description

### FIELD OF THE INVENTION

The present invention relates to methods and kits for diagnosing systemic lupus erythematosus (SLE) in a subject. Particularly, the present invention relates to a specific antibody profile useful in diagnosing SLE in a subject.

### BACKGROUND OF THE INVENTION

Systemic lupus erythematosus (SLE) is an inflammatory disorder of autoimmune etiology, occurring predominantly in young women. SLE can affect many of the body's organ systems, including kidneys, skin, joints, nervous system, serous membranes, blood cells and vessels. Although the specific cause of SLE is unknown, multiple factors are associated with the development of the disease, including genetic, racial, hormonal, and environmental factors.

SLE course is usually chronic, relapsing, and unpredictable. Untreated SLE can be fatal as it progresses from attack of skin and joints to internal organs, including lung, heart, and kidneys, thus making early and accurate diagnosis of and/or assessment of risk of developing SLE particularly critical. SLE mainly appears as a series of flare-ups, with intervening periods of little or no disease manifestation. Kidney damage, measured by the amount of protein in the urine, is one of the most acute areas of damage associated with pathogenicity in SLE, and accounts for at least 50% of the mortality and morbidity of the disease.

SLE is characterized by the production of unusual autoantibodies in the blood. Over 100 different self-molecules have been found to bind autoantibodies in different patients (Sherer et al., 2004, Semin. Arthritis. Rheum. 34:501-37), forming immune complexes which circulate the blood and eventually deposit in tissues. These immune complex depositions cause chronic inflammation and eventually tissue damage. The autoantibodies also have direct pathogenic effects contributing to hemolytic anemia and thrombocytopenia.

Typically, a diagnosis of SLE can be made on the basis of eleven criteria defined by the American College of Rheumatology (ACR). These criteria include malar rash, discoid rash, photosensitivity, oral ulcers, arthritis, serositis, renal disorder, neurologic disorder, hematologic disorder (e.g., leucopenia, lymphopenia, hemolytic anemia or thrombocytopenia), immunologic disorder and anti-nuclear antibodies (ANA) (Tan et al., 1997, Arthritis Rheum 1997, 40:1725). A subject can be clinically diagnosed with SLE if he meets at least four of the eleven criteria. Nevertheless, SLE is still possible even in case when less then four criteria are present.

While anti-nuclear antibodies and autoantibodies to dsDNA, phospholipids and Sm proteins are among the eleven criteria used for diagnosing SLE (Tan et al., 1997, Arthritis Rheum 1997, 40:1725), many patients diagnosed with SLE lack these autoantibodies, especially when they are in clinical remission.

One of the most difficult challenges in clinical management of complex autoimmune diseases such as SLE is the accurate and early identification of the disease in a patient. In addition, no reliable diagnostic markers have been identified that enable clinicians or others to accurately define pathophysiological aspects of SLE, clinical activity, response to therapy, or prognosis.

### The antigen chip

Antigen microarrays are recently developed tools for the high-throughput characterization of the immune response (Robinson et al., 2002, Nat Med 8, 295-301), and have been used to analyze immune responses in vaccination and in autoimmune disorders (Robinson et al., 2002; Robinson et al., 2003, Nat Biotechnol. 21, 1033-9; Quintana et al., 2004; Kanter et al., 2006, Nat Med 12, 138-43). It has been hypothesized, that patterns of multiple reactivities may be more revealing than single antigen-antibody relationships (Quintana et al., 2006, Lupus 15, 428-30) as shown in previous analyses of autoimmune repertoires of mice (Quintana et al., 2004; Quintana et al., 2001, J Autoimmun 17, 191-7) and humans (Merbl et al., 2007, J Clin Invest 117, 712-8; Quintana et al., 2003, J Autoimmun 21, 65-75) in health and disease. Thus, autoantibody repertoires have the potential to provide both new insights into the pathogenesis of the disease and to serve as immune biomarkers (Cohen, 2007, Nat Rev Immunol. 7, 569-74) of the disease process.

PCT Pub. No. WO 02/08755 to some of the inventors of the present invention is directed to a method, system and an article of manufacture for clustering and thereby identifying predefined antigens reactive with undetermined immunoglobulins of sera derived from patient subjects in need of diagnosis of disease or monitoring of treatment. The '755 publication discloses the use of antigen arrays for identifying antigens reactive with immunoglobulins of sera derived from subjects afflicted with various diseases. Further disclosed are diagnostic methods, and systems useful in these methods, employing the step of clustering a subset of antigens of a plurality of antigens, said subset of antigens being reactive with a plurality of antibodies being derived from a plurality of patients having an impaired immune system and suffering from a disease, and associating or deassociating the antibodies of a subject with the resulting cluster.

U.S. Pat. App. Pub. No. 2005/0260770 to some of the inventors of the present invention discloses an antigen array system and diagnostic uses thereof. The application provides a method of diagnosing an immune disease, and particularly type 1 diabetes, or a predisposition thereto in a subject, comprising determining a capacity of immunoglobulins of the subject to specifically bind each antigen probe of an antigen probe set.

PCT Pub. No. WO 10/128506 to some of the inventors of the present invention relates to methods of identifying the development of a cardiovascular disease in an individual, specifically, for recognizing the development of an acute myocardial infarction (AMI) process in an individual.Li et al. disclose in Protein Clinical and Experimental Immunology 147 (2006), 60-70 protein array auto-antibody profiles for insights into SLE and incomplete lupus syndromes.

In the article "Autoantigen microarray for multiplex characterization of auto-antibody responses" published in Nature Medicine 8(3) (2002), 295-301, Robinson et al. discuss the use of microarrays containing autoantigens for the detection of antibodies recognizing said autoantibodies involved in different diseases, such as systemic lupus erythematosus and rheumatoid arthritis.

None of the prior art discloses an antigen array that can provide a specific, reliable, accurate and discriminatory assay for diagnosing SLE. Such discriminatory assays would be highly valuable in identification of the presence of SLE in patients or the determination of susceptibility to develop the disease and in tailoring adequate therapeutic approach for each patient.

There remains a need for improved diagnostic methods and kits useful in diagnosing SLE in a subject.

### SUMMARY OF THE INVENTION

The present invention provides methods for diagnosing systemic lupus erythematosus (SLE) in a subject.

The present invention is based in part on the unexpected results obtained when testing the antibody reactivity of SLE patients using an antigen array. The analysis resulted in the identification of unique autoantibody reactivity patterns. Unexpectedly, the unique autoantibody patterns persist independently of disease activity and are present also in subjects with long-term clinical remission (e.g., subjects in renal remission). Advantageously, the reactivity pattern showed strikingly high sensitivity and high specificity for SLE. Furthermore, a healthy control subject who had the SLE antibody pattern was later found to develop clinical SLE.

Thus, the present invention provides unique antigen-autoantibody reactivity patterns relevant to SLE. While several single antigens (e.g., hyaluronic acid) were identified as being sufficient on their own to adequately diagnose SLE, specific combinations of these antigens, as detailed in Table 1 herein below, were significantly more accurate and reliable in discriminating SLE patients and control subjects than each antigen alone.

**Table 1 - Antigens discriminating SLE and healthy controls**

| **Antigen** | **SEQ ID NO:** | **CAS number** |
|---|---|---|
| Hyaluronic acid | | 9067-32-7 |
| CD99 | SEQ ID NO: 1 | |
| double strand DNA (dsDNA) | | 73049-39-5 |
| single strand DNA (ssDNA) | | 91080-16-9 |
| Epstein-Barr virus (EBV) | SEQ ID NO: 2 | |
| Myeloperoxidase (MPO) | SEQ ID NO: 3 | |
| Insulin-like growth factor binding protein-1 (IGFBP-1) | SEQ ID NO: 4 | |
| Cardiolipin | | 383907-10-6 |
| Collagen Type III (Collagen III) | | 9007-34-5 |
| Collagen Type IV (Collagen IV) | | 9007-34-5 |
| actin | | 51005-14-2 |
| Bone morphogenetic protein-4 (BMP4) | SEQ ID NO: 5 | |
| CMV (CMV Pp150) | SEQ ID NO: 6 | |
| F50 | SEQ ID NO: 7 | |
| Hepatocyte Growth Factor (HGF) | SEQ ID NO: 8 | |
| | | |
| HSP60p18 | SEQ ID NO: 9 | |
| Rubella virus (RV) | SEQ ID NO: 10 | |
| S100 calcium-binding protein A4 (S100A4) | SEQ ID NO: 11 | |
| CITED 1 | SEQ ID NO: 12 | |
| Fragile Histidine Triad (FHIT) | SEQ ID NO: 13 | |

According to a first aspect, the present invention provides a method of diagnosing systemic lupus erythematosus (SLE) in a subject, the method comprising:
(i) determining the reactivity of IgG and IgM antibodies in a sample obtained from the subject to at least four antigens selected from the group consisting of: IGFBP1, CD99, hyaluronic acid, EBV, ssDNA, dsDNA, MPO, cardiolipin, collagen III, collagen IV, actin, BMP4, CMV, F50, HGF, HSP60p18, RV, S100A4, CITED 1 and FHIT, thereby determining the reactivity pattern of the sample to the plurality of antigens, and
(ii) comparing the reactivity pattern of said sample to a control reactivity pattern, wherein an increased reactivity of IgG antibodies to a plurality of antigens selected from the group consisting of: hyaluronic acid, EBV, ssDNA, dsDNA, BMP4, F50, HGF and HSP60p18, and/or a decreased reactivity of IgM antibodies to a plurality of antigens selected from the group consisting of: IGFBP1, CD99, MPO, cardiolipin, collagen III, collagen IV, actin, CMV, RV, S100A4, CITED1 and FHIT compared to the reactivity pattern of a control sample is an indication that the subject is afflicted with SLE.

In one embodiment, the antigens comprise at least five antigens. In another embodiment, the antigens comprise at least six antigens. In another embodiment, the antigens comprise at least seven antigens. In another embodiment, the antigens comprise at least eight antigens. According to an exemplary embodiment the antigens are IGFBP1, CD99, hyaluronic acid, EBV, ssDNA, dsDNA, MPO, cardiolipin and collagen III. Each possibility represents a separate embodiment of the invention.

As used herein, the "reactivity of antibodies in a sample" to "a plurality of antigens" refers to the immune reactivity of each antibody in the sample to a specific antigen selected from the plurality of antigens. The immune reactivity of the antibody to the antigen, i.e. its ability to specifically bind the antigen, may be used to determine the amount of the antibody in the sample. Specifically, determining the "reactivity of IgG and IgM antibodies in a sample" refers to the reactivity of at least one IgG antibody in the sample to a specific antigen selected from the plurality of antigens, and at least one IgM antibody to another specific antigen selected from the plurality of antigens.

The reactivity pattern of the sample thus reflects the levels of each one of the tested antibodies in the sample, thereby providing a quantitative assay. In a particular embodiment, the reactivity is quantitatively determined. Thus, for instance, the reactivity of an antibody to an antigen may be increased or decreased. In certain embodiments, the reactivity of at least one antibody to a specific antigen (from the plurality of antigens) is up-regulated. Preferably, the reactivity of the IgG antibodies is up-regulated (i.e., increased). In additional embodiments, the reactivity of at least one antibody to a specific antigen is down-regulated. Preferably, the reactivity of the IgM antibodies is down-regulated (i.e., decreased).

Typically, determining the reactivity of antibodies in the sample to the plurality of antigens is performed using an immunoassay. Advantageously, the plurality of antigens may be used in the form of an antigen array.

According to an embodiment, the method comprises determining the reactivity of IgG antibodies in the sample obtained from the subject to a plurality of antigens selected from the group consisting of: hyaluronic acid, EBV, ssDNA, dsDNA, BMP4, F50, HGF and HSP60p18. According to an exemplary embodiment, the method comprises determining the reactivity of IgG antibodies in the sample obtained from the subject to a plurality of antigens selected from the group consisting of: hyaluronic acid, EBV, ssDNA and dsDNA. In a specific embodiment, the reactivity of an IgG antibody to an antigen (selected from the plurality of antigens) is up-regulated. According to these embodiments, an up-regulation between the reactivity pattern of said sample obtained from the subject compared to the reactivity pattern of a control sample is an indication that the subject is afflicted with SLE.

The method comprises determining the reactivity of IgM antibodies in the sample obtained from the subject to a plurality of antigens selected from the group consisting of: CD99, IGFBP1, MPO, cardiolipin, Collagen III, collagen IV, actin, CMV, RV, S100A4, CITED1 and FHIT. According to an exemplary embodiment, the method comprises determining the reactivity of IgM antibodies in the serum sample obtained from the subject to a plurality of antigens selected from the group consisting of: CD99, MPO, IGFBP1, cardiolipin and Collagen III. According to another embodiment, the method comprises determining the reactivity of IgM antibodies in the serum sample obtained from the subject to a plurality of antigens selected from the group consisting of: CD99, IGFBP1, MPO and cardiolipin. According to yet another embodiment, the method comprises determining the reactivity of IgM antibodies in the serum sample obtained from the subject to a plurality of antigens selected from the group consisting of: CD99, MPO and Collagen III. In a specific embodiment, the reactivity of an IgM antibody to an antigen (from the plurality of antigens) is down-regulated. In a particular embodiment, a down regulation between the reactivity pattern of said sample obtained from the subject compared to the control reactivity pattern is an indication that the subject is afflicted with SLE.

According to another embodiment, the method comprises determining the reactivity of IgM antibodies in the serum sample obtained from the subject to a plurality of antigens selected from the group consisting of: CD99, MPO and Collagen III. In a particular embodiment, a down regulation between the reactivity pattern of said sample obtained from the subject compared to the control reactivity pattern is an indication that the subject is afflicted with SLE in renal remission.

According to another embodiment, the sample obtained from the subject is a biological fluid. According to some embodiments, the sample is selected from the group consisting of plasma, serum, blood, cerebrospinal fluid, synovial fluid, sputum, urine, saliva, tears, lymph specimen, or any other biological fluid known in the art. According to certain embodiments, the sample obtained from the subject is selected from the group consisting of plasma, serum and blood. According to one embodiment, the sample is a serum sample. Each possibility represents a separate embodiment of the invention.

According to certain embodiments of the methods of the present invention, the control is selected from the group consisting of a sample from at least one healthy individual, a panel of control samples from a set of healthy individuals, and a stored set of data from healthy individuals. Typically, a healthy individual is a subject not afflicted with SLE or any other form of lupus. In another embodiment, a healthy individual is a subject not afflicted with an autoimmune disease.

According to some embodiments, the method further comprises diluting the sample e.g. 1:10 or more before determining the reactivity of antibodies in the sample.

A "difference" between reactivity patterns refers, in different embodiments, to a statistically significant difference, or in other embodiments to a difference as recognized by a skilled artisan.

Advantageously, the methods of the invention may employ the use of learning and pattern recognition analyzers, clustering algorithms and the like, in order to discriminate between reactivity patterns of healthy control subjects to those of patients having SLE. As such, this term specifically includes a difference measured by, for example, determining the reactivity of antibodies in a test sample to a plurality of antigens, and comparing the resulting reactivity pattern to the reactivity patterns of negative and positive control samples (e.g. samples obtained from control subjects which are not afflicted with SLE or patients afflicted with SLE, respectively) using such algorithms and/or analyzers. The difference may also be measured by comparing the reactivity pattern of the test sample to a predetermined classification rule obtained in such manner.

According to another aspect, the antigens are selected from the group consisting of: IGFBP1, CD99, hyaluronic acid, MPO, collagen III.

According to one embodiment, the antigens further comprise at least one, at least two or at least three antigens selected from dsDNA, ssDNA, EBV and cardiolipin. Each possibility represents a separate embodiment of the invention.

Other objects, features and advantages of the present invention will become clear from the following description and drawings.

### BRIEF DESCRIPTION OF THE DRAWINGS

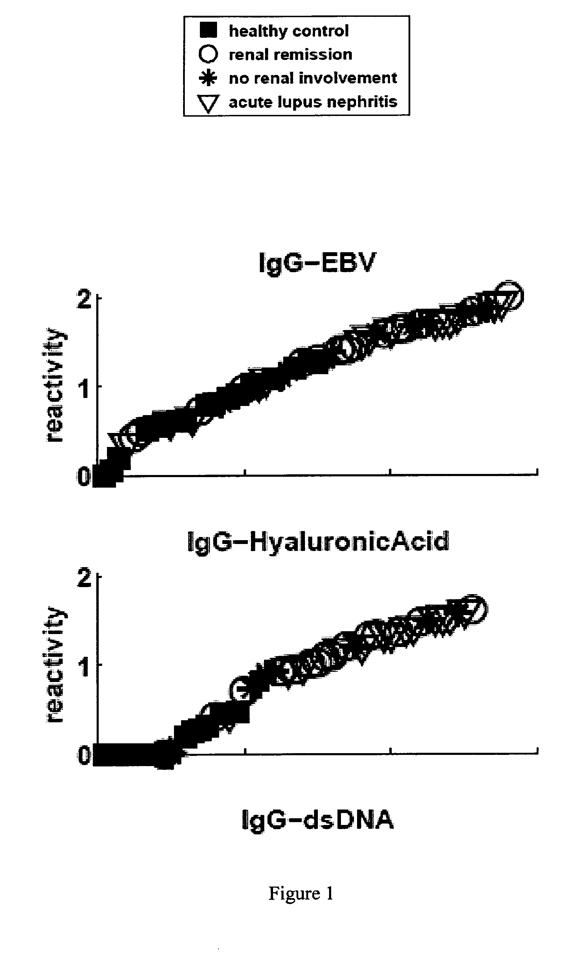

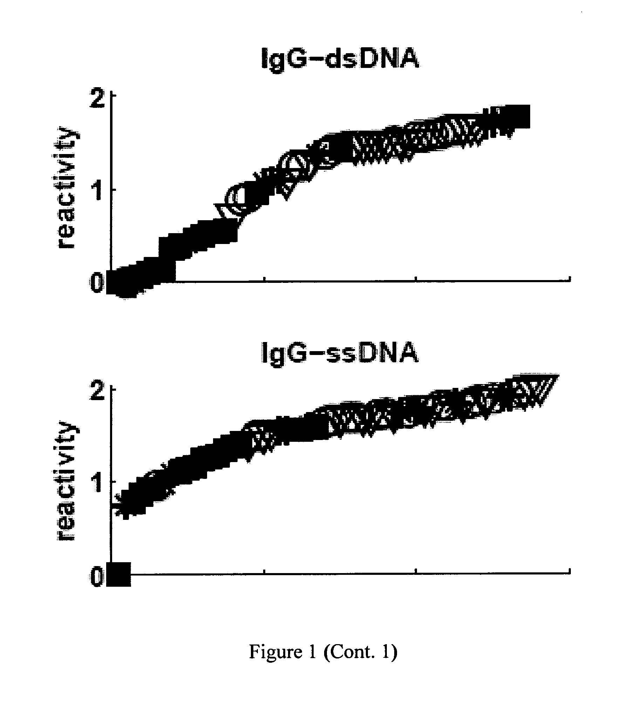

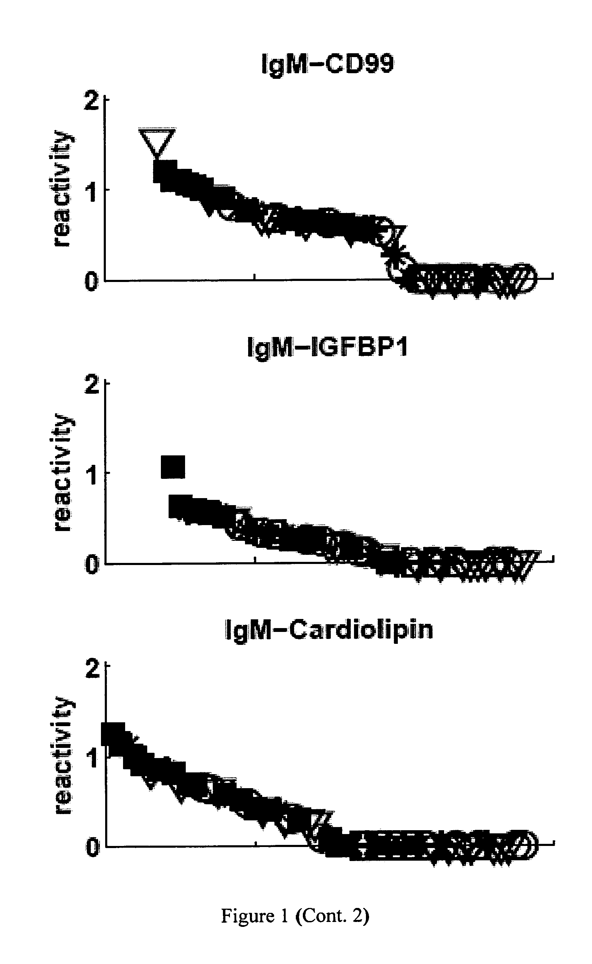

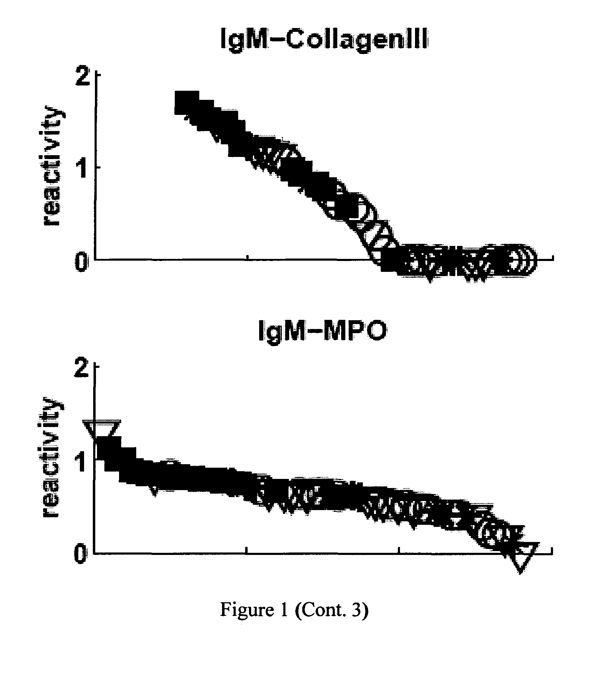

**Figure 1** shows the antibody reactivity of individual subjects to the antigen reactivities that characterize patients with SLE. Sera from healthy controls (black squares) and from SLE subjects in renal remission (open circles), acute lupus nephritis (open triangles), or without renal involvement (asterisk) were tested for antibody reactivities to the designated antigen. The relative amount of antibody reactivity is shown on the Y axis. The X axis orders the subjects according to their relative reactivity.

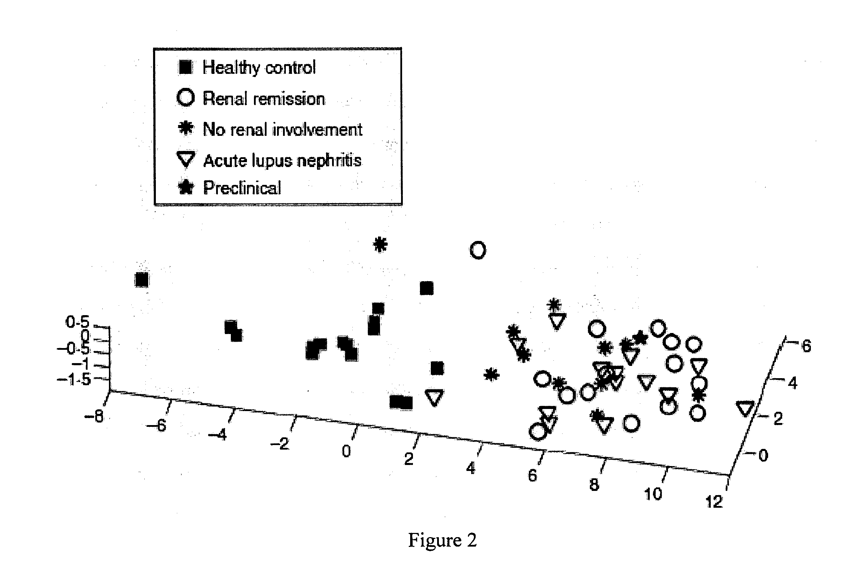

**Figure 2** depicts a three-dimensional principal component analysis (PCA). The PCA was based on the seven antigen reactivities that distinguish healthy controls from patients with SLE in renal remission (Table 6). The star represents the signature of a subject who was healthy at the time of serum collection but who later developed SLE, healthy controls are represented as black squares, SLE subjects in renal remission are depicted as open circles, subjects with acute lupus nephritis are depicted as triangles and SLE subjects without renal involvement are represented as asterisk.

### DETAILED DESCRIPTION OF THE INVENTION

The present invention provides methods of diagnosing systemic lupus erythematosus (SLE) in a subject using antigen probe arrays for practicing such a diagnosis, and identifies specific antigen probe sets for generating such arrays. Hence, an autoantibody-based biomarker test for early diagnosis of SLE, including SLE in renal remission is disclosed.

Without wishing to be bound by any particular theory or mechanism of action, the invention is based in part on the finding that the antibody reactivity profile in serum of SLE patients was clearly distinct of healthy control individuals. Although serum autoantibodies have been extensively investigated in SLE, the unique antibody immune signatures as described herein have not been described before. Advantageously, the unique antibody signatures of the present invention provide highly sensitive and specific assays for diagnosing SLE.

According to some embodiments, the present invention provides a method of diagnosing systemic lupus erythematosus (SLE) in a subject, the method comprising:
(i) determining the reactivity of IgG and IgM antibodies in a sample obtained from the subject to at least four antigens selected from the group consisting of: IGFBP1, CD99, hyaluronic acid, EBV, ssDNA, dsDNA, MPO, cardiolipin, collagen III, collagen IV, actin, BMP4, CMV, F50, HGF, HSP60p18, RV, S100A4, CITED 1 and FHIT, thereby determining the reactivity pattern of the sample to the plurality of antigens, and
(ii) comparing the reactivity pattern of said sample to a control reactivity pattern,
wherein an increased reactivity of IgG antibodies to a plurality of antigens selected from the group consisting of: hyaluronic acid, EBV, ssDNA, dsDNA, BMP4, F50, HGF and HSP60p18, and/or a decreased reactivity of IgM antibodies to a plurality of antigens selected from the group consisting of: IGFBP1, CD99, MPO, cardiolipin, collagen III, collagen IV, actin, CMV, RV, S100A4, CITED1 and FHIT compared to the reactivity pattern of a control sample is an indication that the subject is afflicted with SLE.

The reactivity pattern of the sample reflects the levels of each one of the tested antibodies in the sample, thereby providing a quantitative assay. In a preferred embodiment, the antibodies are quantitatively determined. In yet another preferred embodiment, a quantitative difference between the reactivity pattern of said sample obtained from the subject compared to the control reactivity pattern is an indication that the subject is afflicted with SLE. The up-regulation of the reactivity of an antibody, specifically an IgG antibody, in a sample to an antigen refers to an increase (i.e., elevation) of about at least two, about at least three, about at least four, or about at least five times higher (i.e., greater) than the reactivity levels of the antibody to the antigen in the control. The down-regulation of the reactivity of an antibody, specifically an IgM antibody, in a sample to an antigen refers to a decrease (i.e., reduction) of about at least two, about at least three, about at least four, or about at least five times lower than the reactivity levels of the antibody to the antigen in the control.

As exemplified herein below, antigen analysis of autoantibodies (e.g., using microarray analysis) can identify serum autoantibody patterns associated with different clinical forms of SLE; the signatures were based on collective autoantibody patterns, not single autoantibody reactivities. These informative patterns included IgG autoantibodies as well as IgM autoantibodies. Moreover, the informative patterns included decreases as well as increases of autoantibody reactivities relative to those found in healthy controls.

In a particular embodiment, the method comprises:
(i) determining the reactivity of IgG antibodies in a sample obtained from the subject to a plurality of antigens selected from the group consisting of: hyaluronic acid, EBV, ssDNA and dsDNA;
(ii) determining the reactivity of IgM antibodies in a sample obtained from the subject to a plurality of antigens selected from the group consisting of: CD99, MPO, IGFBP1, cardiolipin and Collagen III; thereby determining the reactivity pattern of the sample to the plurality of antigens; and
(iii) comparing the reactivity pattern of said sample to a control reactivity pattern, wherein an increase between the reactivity pattern of the IgG antibodies in said sample obtained from the subject compared to the control reactivity pattern is an indication that the subject is afflicted with SLE, or wherein a decrease between the reactivity pattern of the IgM antibodies in said sample obtained from the subject compared to the control reactivity pattern is an indication that the subject is afflicted with SLE.

In yet another embodiment, an increase between the reactivity pattern of the IgG antibodies in said sample obtained from the subject compared to the control reactivity pattern is an indication that the subject is afflicted with SLE, and a decrease between the reactivity pattern of the IgM antibodies in said sample obtained from the subject compared to the control reactivity pattern is an indication that the subject is afflicted with SLE.

### Antigen probes and antigen probe sets

The present specification also discloses antigen probes and antigen probe sets useful for diagnosing SLE, as detailed herein.

According to the principles of the invention, the invention further provides a plurality of antigens also referred to herein as antigen probe sets. These antigen probe sets comprising a plurality of antigens are reactive specifically with the sera of subjects having SLE. According to the principles of the invention, the plurality of antigens may advantageously be used in the form of an antigen array. According to some embodiments the antigen array is conveniently arranged in the form of an antigen chip.

A "probe" as used herein means any compound capable of specific binding to a component. According to one aspect, the present invention provides an antigen probe set comprising a plurality of antigens selected from the group consisting of: CD99, IGFBP1, hyaluronic acid, EBV, ssDNA, dsDNA, MPO, cardiolipin, collagen III, collagen IV, actin, BMP4, CMV, F50, HGF, HRP, HSP60p18, RV, S100A4, CITED1 and FHIT. According to certain embodiments, the antigen probe set comprises a subset of the antigens of the present invention. In a particular embodiment, the subset of antigen consists of CD99, IGFBP1, hyaluronic acid, EBV, ssDNA, dsDNA, MPO, cardiolipin and collagen III. The reactivity of antibodies to the plurality of antigens of the invention may be determined according to techniques known in the art. Further, the antigens used in the present invention are known in the art and are commercially available, e.g., from Sigma Aldrich or Prospec.

### Hyaluronic acid

Hyaluronic acid is an anionic, nonsulfated glycosaminoglycan distributed widely throughout connective, epithelial, and neural tissues. In a particular embodiment, the hyaluronic acid antigen of the present invention is a human hyaluronic acid, e.g. from the umbilical cord of human placenta (commercially available, e.g., from Sigma Aldrich, catalog number H1876; CAS number 9067-32-7). Additional non limiting examples of hyaluronic acid have a CAS number selected from: 31799-91-4 or 9067-32-7.

### EBV

The Epstein-Barr virus (EBV), also called Human herpes virus 4 (HHV-4), is a virus of the herpes family. The reactivity of antibodies to the EBV antigen may be determined according to techniques known in the art. In a particular embodiment, the EBV antigen of the present invention contains the HHV-4 Early Antigen Type D (GeneBank: CAD53407.1), C-terminus regions residing at amino acids 306-390 as set forth in SEQ ID NO: 2. In one embodiment, said EBV consists of SEQ ID NO: 2, or a analog or fragment thereof. The EBV antigen is commercially available, e.g., from Prospec, catalog number CMV-272.

### Double strand deoxyribonucleic acid (dsDNA)

Anti-dsDNA antibodies are highly specific for SLE; they are present in 70% of cases, whereas they appear in only 0.5% of people without SLE. The reactivity of antibodies to the dsDNA antigen may be determined according to techniques known in the art. In a particular embodiment, dsDNA has a CAS number of 73049-39-5. The dsDNA antigen is commercially available, e.g., from Sigma Aldrich, catalog number D1501.

### Single strand deoxyribonucleic acid (ssDNA)

The reactivity of antibodies to the ssDNA antigen may be determined according to techniques known in the art. In a particular embodiment, ssDNA has a CAS number of 91080-16-9. The ssDNA antigen is commercially available, e.g., from Sigma Aldrich, catalog number D8899.

### CD99

CD99 (also known as E2 antigen, T-cell surface glycolprotein) is expressed on the cell membrane of some lymphocytes, cortical thymocytes, and granulosa cells of the ovary. The antigen is also expressed by most pancreatic islet cells, Sertoli cells of the testis, and some endothelial cells. In a particular embodiment, the CD99 antigen of the present invention is a human CD99 (NP_002405 or NP_001116370.1). The amino acid sequence of human CD99 isoform a precursor is set forth in SEQ ID NO:1. In one embodiment, said CD99 antigen consists of SEQ ID NO: 1, or an analog or fragment thereof. Said CD99 antigen is commercially available, e.g., from Prospec, catalog number PRO-294.

### Myeloperoxidase (MPO)

MPO is an important enzyme used by granulocytes during phagocytic lysis of foreign particles engulfed. In normal tissues and in a variety of myeloproliferative disorders myeloid cells of both neutrophilic and eosinophilic types, at all stages of maturation, exhibit strong cytoplasmic reactivity for MPO. In a particular embodiment, the MPO antigen of the present invention is a human MPO (NP_000241.1). The amino acid sequence of human MPO is set forth in SEQ ID NO:3. In one embodiment, said MPO antigen consists of SEQ ID NO: 3, or an analog or fragment thereof. Said MPO antigen is commercially available, e.g., from Prospec, catalog number ENZ-334.

### Insulin-like growth factor binding_protein (IGFBP1)-1

IGFBP1 is a member of the insulin-like growth factor binding protein (IGFBP) family and encodes a protein with an IGFBP domain and a thyroglobulin type-I domain. The protein binds both insulin-like growth factors (IGFs) I and II and circulates in the plasma. Human IGFBP1 may be recombinantly produced as a single polypeptide chain containing 218 amino acids, having a molecular mass of 28.8 KDa. In a particular embodiment, the IGFBP1 antigen of the present invention is a human IGFBP1 (NP_000587.1)). The amino acid sequence of human IGFBP1 is set forth in SEQ ID NO:4. In one embodiment, said IGFBP1 antigen consists of SEQ ID NO: 4, or an analog or fragment thereof. Said IGFBP1 antigen is commercially available, e.g., from Prospec, catalog number CYT-299.

### Cardiolipin

Cardiolipin (1,3-bis(sn-3'-phosphatidyl)-sn-glycerol) is a mitochondrial phospholipid that is found in mammalian tissues in low concentrations, most often in the inner mitochodrial membrane. Cardiolipin inhibits cell attachment of fibronectin, vitronectin and Type I collagen. In a particular embodiment, the cardiolipin antigen of the present invention is a bovine cardiolipin. The reactivity of antibodies to the cadiolipin antigen may be determined according to techniques known in the art. In a particular embodiment, cadiolipin has a CAS number of 383907-10-6. The bovine cardiolipin antigen is commercially available, e.g., from Sigma Aldrich, catalog number C0563.

### Collagen Type III

Type III collagen is the second most abundant collagen in human tissues and occurs particularly in tissues exhibiting elastic properties, such as skin, blood vessels and various internal organs. Mutations of type III collagen cause the most severe form of Ehlers-Danlos syndrome, EDS IV, which affect arteries, internal organs, joints and skin, and may cause sudden death when the large arteries rupture. In a particular embodiment, the type III collagen antigen of the present invention is a Bornstein and Traub Type III collagen, e.g., from human placenta. The reactivity of antibodies to the collagen-III antigen may be determined according to techniques known in the art. In a particular embodiment, collagen-III has a CAS number of 9007-34-5. The collagen-III antigen is commercially available, e.g., from Sigma Aldrich, catalog number C4407.

### Collagen Type IV

Unlike most collagens, type IV collagen is an exclusive member of the basement membranes and through a complex inter- and intramolecular interactions form supramolecular networks that influence cell adhesion, migration, and differentiation (Khoshnoodi et al., Microsc Res Tech. 2008 May;71(5):357-70). In a particular embodiment, the type IV collagen antigen of the present invention is a Bornstein and Traub Type IV collagen, e.g., from human placenta. The reactivity of antibodies to the collagen-IV antigen may be determined according to techniques known in the art. In a particular embodiment, collagen-IV has a CAS number of 9007-34-5. The collagen-IV antigen is commercially available, e.g., from Sigma Aldrich, catalog number C7521.

### Actin

Actin is a 43 kDa protein that is very highly conserved between species. There are three main actin isotypes (α, β and y), which show >90% amino-acid (aa) homology between isotypes and >98% homology within members of a particular isotypic group. Actin participates in many important cellular processes including muscle contraction, cell motility, cell division and cytokinesis, vesicle and organelle movement, cell signaling, and the establishment and maintenance of cell junctions and cell shape. In a particular embodiment, the actin antigen of the present invention is a bovine actin. The reactivity of antibodies to the actin antigen may be determined according to techniques known in the art. In a particular embodiment, actin has a CAS number of 51005-14-2. The bovine actin antigen is commercially available, e.g., from Sigma Aldrich, catalog number A3653.

### Bone morphogenetic protein-4 (BMP4)

BMP4 plays an important role in the onset of endochondral bone formation in humans. A reduction in BMP4 expression has been associated with a variety of bone diseases, including the heritable disorder Fibrodysplasia Ossificans Progressiva. Human BMP4 (e.g., NP_570912.2) may be recombinant produced in E.Coli as a monomeric, non-glycosylated, polypeptide chain (molecular mass of 13009 Dalton). In a particular embodiment, the BMP4 antigen of the present invention is a human BMP4 (commercially available, e.g., from Prospec, catalog number CYT-361). In a specific embodiment, the BMP4 antigen has the amino acid sequence as set forth in SEQ ID NO: 5 (SPKHHSQRARKKNKNCRRHSLYVDFSDVGWNDWIVAPPGYQAFYCHGDCPFPL ADHLNSTNHAIVQTLVNSVNSSIPKACCVPTELSAISMLYLDEYDKVVLKNYQEMV VEGCGCR, or an analog or fragment thereof.

### CMV

CMV (CytomegaloVirus) belongs to the Betaherpesvirinae subfamily of Herpesviridae which includes herpes simplex virustypes 1 and 2, varicella-zoster virus, and Epstein-Barrvirus. CMV is a double-stranded linear DNA virus with 162 hexagonal protein capsomeres surrounded by a lipid membrane. CMV has the largest genome of the herpes viruses, ranging from 230-240 kilobase pairs. Human CMV is composed of unique and inverted repeats that include the existence of 4 genome isomers caused by inversion of L-S genome components (class E). The CMV antigen used in the examples herein below is an E.Coli derived recombinant protein containing the CMV Pp150 (UL32) immunodominant regions residing at amino acids 1011-1048. Said CMV Pp150 (e.g., GI:224496137 or GI:224496135) is known to those skilled in the art. The amino acid sequence of said CMV Pp150 is set forth in SEQ ID NO:6. In one embodiment, said CMV Pp150 consists of SEQ ID NO: 6, or an analog or fragment thereof. The CMV antigen of the present invention is commercially available, e.g., from Prospec, catalog number CMV-216.

### F50

The F50 antigen used in the examples herein below is an oligopeptide having the amino acid sequence CVKGGTTKIFLVGDYSSSAE as set forth as SEQ ID NO: 7. In one embodiment, said F50 antigen consists of SEQ ID NO: 7, or an analog or fragment thereof.

### Hepatocyte Growth Factor (HGF)

HGF is a multifunctional growth factor which regulates both cell growth and cell motility. It exerts a strong mitogenic effect on hepatocytes and primary epithelial cells. Human HGF may be recombinantly produced in Baculovirus as a heterodimer, non-glycosylated, polypeptide chain consisting an α-chain of 463 amino acids and β-chain of 234 amino acids having a total molecular mass of 78.0 KDa. In a particular embodiment, the HGF antigen of the present invention is a human HGF (AAA64297.1). The amino acid sequence of said human HGF is set forth in SEQ ID NO:8. In one embodiment, said HGF consists of SEQ ID NO: 8, or an analog or fragment thereof. Said HGF antigen is commercially available, e.g., from Prospec, catalog number CYT-244.

### Horseradish peroxide (HRP)

Horseradish peroxidase is a single chain polypeptide containing four disulfide bridges. It is a glycoprotein containing 18% carbohydrate. The carbohydrate composition consists of galactose, arabinose, xylose, fucose, mannose, mannosamine, and galactosamine depending upon the specific isozyme. HRP belongs to the ferroprotoporphyrin group of peroxidases and may be isolated from horseradish roots (*Amoracia rusticana*). The reactivity of antibodies to the HRP antigen may be determined according to techniques known in the art. In a particular embodiment, HRP has a CAS number of 9003-99-0. The HRP antigen is commercially available, e.g., from Sigma Aldrich, catalog number P6782.

### HSP60p18

The HSP60p18 antigen used in the examples herein below is an oligopeptide having the amino acid sequence QSIVPALEIANAHRKPLVIIA as set forth as SEQ ID NO: 9. In one embodiment, said HSP60p18 antigen consists of SEQ ID NO: 9, or an analog or fragment thereof.

### Rubella virus (RV)

RV is an enveloped positive-strand RNA virus of the family Togaviridae. In a specific embodiment, the RV antigen is an E.Coli derived recombinant protein containing the RV Capsid C regions, amino acids 1-123. The amino acid sequence of the RV Capsid C is known to those skilled in the art, e.g., NP_740662.1. The amino acid sequence of amino acids 1-123 of RV capsid c is set forth in SEQ ID NO:10. In one embodiment, said RV antigen consists of SEQ ID NO: 10, or an analog or fragment thereof. Said RV antigen is commercially available, e.g., from Prospec, catalog number RUB-293.

### S100A4

S100A4 (also known as S100 calcium-binding protein A4, Metastasin, Calvasculin) is a member of the S100 family of proteins containing 2 EF-hand calcium-binding motifs. S100A is composed of an alpha and beta chain. S100A4 may function in motility, invasion, and tubulin polymerization. In a specific embodiment, the S100A4 antigen of the invention is a human S100A4 (CAG29341.1). The amino acid sequence of human S100A4 is set forth in SEQ ID NO: 11. In one embodiment, said S100A4 antigen consists of SEQ ID NO: 11, or an analog or fragment thereof. Said S100A4 antigen is commercially available, e.g., from Prospec, catalog number PRO-307.

### CITED 1

The human Cbp/p300-interacting transactivator 1 (CITED1, also known as Melanocyte-specific protein 1 or MSG1) is a protein having 193 amino acids (NP_001138359.1). The amino acid sequence of human CITED1 is set forth in SEQ ID NO:12. In one embodiment, said CITED1 antigen consists of SEQ ID NO: 12, or an analog or fragment thereof. CITED1 antigen is commercially available, e.g., from Prospec, catalog number PRO-295.

### FHIT

FHIT (Fragile Histidine Triad) is a member of the histidine triad gene family, involved in purine metabolism. The FHIT protein is a tumor suppressor with reduced or no expression in numerous types of cancer. In a specific embodiment, the FHIT antigen of the invention is a human FHIT (ABM66093.1). The amino acid sequence of human FHIT is set forth in SEQ ID NO:13. In one embodiment, said FHIT antigen consists of SEQ ID NO: 13, or an analog or fragment thereof. Said FHIT antigen is commercially available, e.g., from Prospec, catalog number PRO-297.

The plurality of antigens (or the antigen probe set) comprises or consists of a subset thereof, e.g. at least 4, 5, 6, 7, 8, 9, 10, or 11 different antigens each selected from the antigens disclosed in the present invention. Such subsets may be selected so as to result in optimal sensitivity and/or specificity of the diagnostic assay. In other embodiments, the probe set comprises up to 10, or in other embodiments up to 15, 20, 30, 40 or 50 different antigens. It should be noted, that while such probe sets are considered sufficient for reliably identifying a subject with SLE, the antigen probe sets used in the invention may conveniently be used, in certain embodiments, in the form of antigen arrays comprising a greater number of antigens, e.g. about 25 antigens or more.

According to a particular embodiment, the antigen probe set comprises a plurality of antigens selected from CD99, IGFBP1, hyaluronic acid, MPO, EBV, ssDNA, dsDNA, and cardiolipin, as detailed herein, for the diagnosis of SLE.

In another embodiment, the plurality of antigens consists of hyaluronic acid, EBV, ssDNA and dsDNA. As disclosed herein, this subset of antigens showed up-regulated reactivities with IgG antibodies in serum obtained from subjects with SLE. In another embodiment, the plurality of antigens consists of CD99, MPO, IGFBP1 and cardiolipin. As disclosed herein, this subset of antigens showed down-regulated reactivities with IgM antibodies in serum obtained from subjects with SLE.

In another embodiment, a probe set consisting of the hyaluronic acid, EBV, ssDNA, dsDNA, CD99, MPO and collagen III antigens is sufficient to discriminate between subjects with SLE in renal remission, and healthy individuals that are not afflicted with SLE. In another embodiment, the plurality of antigens consists of hyaluronic acid, EBV, ssDNA and dsDNA. As disclosed herein, this subset of antigens showed up-regulated reactivities with IgG antibodies in serum obtained from subjects with SLE, particularly SLE in renal remission. In another embodiment, the plurality of antigens consists of CD99, MPO and collagen III. As disclosed herein, this subset of antigens showed down-regulated reactivities with IgM antibodies in serum obtained from subjects with SLE, such as in renal remission.

Antigen probes to be used in the assays of the invention may be purified or synthesized using methods well known in the art. For example, an antigenic protein or peptide may be produced using known recombinant or synthetic methods, including, but not limited to, solid phase (e.g. Boc or f-Moc chemistry) and solution phase synthesis methods (Stewart and Young, 1963; Meienhofer, 1973; Schroder and Lupke, 1965; Sambrook *et al*., 2001). One of skill in the art will possess the required expertise to obtain or synthesize the antigen probes of the invention. Some of the antigen probes are also commercially available, e.g. from Sigma (St. Louis, MO, USA), Prospec (Ness-Ziona, Israel), Abnova (Taipei City, Taiwan), Matreya LLC (Pleasant Gap, PA, USA), Avanti Polar Lipids (Alabaster, AL, USA), Calbiochem (San Diego, CA, USA), Chemicon (Temecula, CA, USA), GeneTex (San Antonio, TX, USA), Novus Biologicals (Littleton, CO, USA) Assay Designs (Ann Arbor, MI, USA), ProSci Inc. (Poway, CA, USA), EMD Biosciences (San Diego, CA, USA), Cayman Chemical (Ann Arbor, MI, USA), HyTest (Turku, Finland), Meridian Life Science (Memphis, TN USA) and Biodesign International (Saco, ME, USA), as detailed herein below.

It should be noted, that the invention utilizes antigen probes as well as homologs, fragments and derivatives thereof, as long as these homologs, fragments and derivatives are immunologically cross-reactive with these antigen probes. The term "immunologically cross-reactive" as used herein refers to two or more antigens that are specifically bound by the same antibody. The term "homolog" as used herein refers to a peptide which having at least 70%, at least 75%, at least 80%, at least 85% or at least 90% identity to the antigen's amino acid sequence. Cross-reactivity can be determined by any of a number of immunoassay techniques, such as a competition assay (measuring the ability of a test antigen to competitively inhibit the binding of an antibody to its known antigen).

The term "fragment" as used herein refers to a portion of a polypeptide, or polypeptide analog which remains immunologically cross-reactive with the antigen probes, e.g., to immunospecifically recognize the target antigen. The fragment may have the length of about 40%, about 50%, about 60%, about 70%, about 80%, about 85%, about 90% or about 95% of the respective antigen.

The term peptide typically refers to a polypeptide of up to about 50 amino acid residues in length. According to particular embodiments, the antigenic peptides of the invention may be 10-50 amino acids in length and are typically about 10-30 or about 15-25 amino acids in length.

The term encompasses native peptides (either degradation products, synthetically synthesized peptides, or recombinant peptides), peptidomimetics (typically, synthetically synthesized peptides), and the peptide analogues peptoids and semipeptoids, and may have, for example, modifications rendering the peptides more stable while in a body or more capable of penetrating into cells. Such modifications include, but are not limited to: N-terminus modifications; C-terminus modifications; peptide bond modifications, including but not limited to CH₂-NH, CH₂-S, CH₂-S=O, O=C-NH, CH₂-O, CH₂-CH₂, S=C-NH, CH=CH, and CF=CH; backbone modifications; and residue modifications.

The antigens used in the invention may have a terminal carboxy acid, as a carboxy amide, as a reduced terminal alcohol or as any pharmaceutically acceptable salt, e.g., as metal salt, including sodium, potassium, lithium or calcium salt, or as a salt with an organic base, or as a salt with a mineral acid, including sulfuric acid, hydrochloric acid or phosphoric acid, or with an organic acid e.g., acetic acid or maleic acid.

Functional derivatives consist of chemical modifications to amino acid side chains and/or the carboxyl and/or amino moieties of said peptides. Such derivatized molecules include, for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im-benzylhistidine. Also included as chemical derivatives are those polypeptides, which contain one or more naturally occurring or modified amino acid derivatives of the twenty standard amino acid residues. For example: 4-hydroxyproline may be substituted for proline; 5-hydroxylysine may be substituted for lysine; 3-methylhistidine may be substituted for histidine; homoserine may be substituted or serine; and ornithine may be substituted for lysine.

The amino acid residues described herein are in the "L" isomeric form, unless otherwise indicated. However, residues in the "D" isomeric form can be substituted for any L-amino acid residue, as long as the peptide substantially retains the desired antibody specificity.

Suitable analogs may be readily synthesized by now-standard peptide synthesis methods and apparatus or recombinant methods. All such analogs will essentially be based on the antigens of the invention as regards their amino acid sequence but will have one or more amino acid residues deleted, substituted or added. When amino acid residues are substituted, such conservative replacements which are envisaged are those which do not significantly alter the structure or antigenicity of the polypeptide. For example basic amino acids will be replaced with other basic amino acids, acidic ones with acidic ones and neutral ones with neutral ones. In addition to analogs comprising conservative substitutions as detailed above, analogs comprising non-conservative amino acid substitutions are further contemplated, as long as these analogs are immunologically cross reactive with a peptide of the invention.

The lipid antigens to be used in the assays of the invention may be purified or synthesized using methods well known in the art (see, for example, Biochemistry of Lipids, Lipoproteins, and Membranes, 4.sup.th Ed. (2002; Vance D E and Vance, J E, editors; Elsevier, Amsterdam, Boston); Enzymes in Lipid Modification (2000; Bornsheuer, U T, editor; Wiley-VCH, Weinheim, N.Y.); Lipid Synthesis and Manufacture (1999; Gunstone, F D, editor; Sheffield Academic Press, Sheffield, England; CRC Press, Boca Raton, Fla.); Lipid Biochemistry, 5.sup.th Ed (2002; Gurr, M I, Harwood, J L, and Frayn, K N, editors; Blackwell Science, Oxford, Malden, Mass). In another embodiment, the lipid antigens to be used in the assays of the invention may be commercially purchased as detailed herein below.

### Diagnostic methods

According to some embodiments, the invention provides diagnostic methods useful for the detection of SLE.

According to some embodiments, the methods of the invention are effected by determining the reactivity of antibodies in a sample obtained from a test subject to at least four antigens selected from the group consisting of: hyaluronic acid, CD99, IGFBP1, EBV, ssDNA, dsDNA, MPO, cardiolipin, collagen III, collagen IV, actin, BMP4, CMV, F50, HGF, HSP60p18, RV, S100A4, CITED1 and FHIT, thereby determining the reactivity pattern of the sample to the plurality of antigens, and comparing the reactivity pattern of said sample to a control reactivity pattern. An increased reactivity of IgG antibodies to a plurality of antigens selected from the group consisting of: hyaluronic acid, EBV, ssDNA, dsDNA, BMP4, F50, HGF and HSP60p18, and/or a decreased reactivity of IgM antibodies to a plurality of antigens selected from the group consisting of: IGFBP1, CD99, MPO, cardiolipin, collagen III, collagen IV, actin, CMV, RV, S100A4, CITED1 and FHIT compared to the reactivity pattern of a control sample is an indication that the subject is afflicted with SLE.

As used herein, the "reactivity of antibodies in a sample" to "a plurality of antigens" refers to the immune reactivity of each antibody in the sample to a specific antigen selected from the plurality of antigens. The immune reactivity of the antibody to the antigen, i.e. its ability to specifically bind the antigen, may be used to determine the amount of the antibody in the sample, thereby providing a quantitative assay. The calculated levels of each one of the tested antibodies in the sample are selectively referred to as the reactivity pattern of the sample to these antigens.

An antibody "directed to" an antigen, as used herein is an antibody which is capable of specifically binding the antigen. Determining the levels of antibodies directed to a plurality of antigens includes measuring the level of each antibody in the sample, wherein each antibody is directed to a specific antigen selected from: hyaluronic acid, EBV, ssDNA, dsDNA, CD99, MPO, IGFBP1, cardiolipin, collagen III, collagen IV, actin, BMP4, CMV, F50, HGF, HSP60p18, RV, S100A4, CITED1 and FHIT. This step is typically performed using an immunoassay, as detailed herein.

In other embodiments, determining the reactivity of antibodies in said sample to said plurality of antigens, (and the levels of each one of the tested antibodies in the sample) is performed by a process comprising:
(i) contacting the sample, under conditions such that a specific antigen-antibody complex may be formed, with an antigen probe set comprising said plurality of antigens, and
(ii) quantifying the amount of antigen-antibody complex formed for each antigen probe.

The amount of antigen-antibody complex is indicative of the level of the tested antibody in the sample (or the reactivity of the sample with the antigen).

In certain embodiments, the test sample and control samples comprise IgG and/or IgM antibodies. Particularly, the test sample and control samples may comprise IgG and IgM antibodies. In yet another preferred embodiment, the test and control samples comprise a plurality of IgG antibodies and a plurality of IgM antibodies. In another embodiment, the reactivity of at least one antibody to a specific antigen from the plurality of antigens of the invention is up-regulated. In another embodiment, the reactivity of at least one antibody to a specific antigen is down-regulated.

According to another aspect, the present invention provides a method of assessing whether a subject is at risk of developing SLE, the method comprising: (i) determining the reactivity of IgG and IgM antibodies in a sample obtained from the subject to at least four antigens selected from the group consisting of: hyaluronic acid, CD99, EBV, ssDNA, dsDNA, MPO, IGFBP1, cardiolipin, collagen III, collagen IV, actin, BMP4, CMV, F50, HGF, HSP60p18, RV, S100A4, CITED1 and FHIT, thereby determining the reactivity pattern of the sample to the plurality of antigens, and (ii) comparing the reactivity pattern of said sample to a control reactivity pattern, wherein an increased reactivity of IgG antibodies to a plurality of antigens selected from the group consisting of: hyaluronic acid, EBV, ssDNA, dsDNA, BMP4, F50, HGF and HSP60p18, and/or a decreased reactivity of IgM antibodies to a plurality of antigens selected from the group consisting of: IGFBP1, CD99, MPO, cardiolipin, collagen III, collagen IV, actin, CMV, RV, S100A4, CITED1 and FHIT compared to the reactivity pattern of a control sample is an indication that the subject is afflicted with SLE.

According to an exemplary embodiment the antigens consist of hyaluronic acid, CD99, EBV, ssDNA, dsDNA, MPO, IGFBP1, cardiolipin and collagen III. According to another embodiment the plurality of antigens consist of hyaluronic acid, CD99, MPO, IGFBP1 and collagen III.

In some embodiments, the methods of the present invention employ an antigen microarray system for informatically characterizing informative patterns of antibodies as specific biomarkers for subtypes of SLE, as detailed herein.

Diagnostic methods differ in their sensitivity and specificity. The "sensitivity" of a diagnostic assay is the percentage of diseased individuals who test positive (percent of "true positives"). Diseased individuals not detected by the assay are "false negatives". Subjects who are not diseased and who test negative in the assay are termed "true negatives". The "specificity" of a diagnostic assay is 1 minus the false positive rate, where the "false positive" rate is defined as the proportion of those without the disease who test positive.

In some embodiments, the plurality of antigens is selected to exhibit at least 70%, at least 80%, at least 85%, at least 90%, at least 95%, or at least 99% sensitivity, combined with at least 70%, at least 80%, at least 85%, at least 90%, or at least 95% specificity. In some embodiments, both the sensitivity and specificity are at least 75%, at least 80%, at least 85%, at least 90%, or at least 95%.

In one embodiment, the method distinguishes SLE with a sensitivity of at least 70% at a specificity of at least 85% when compared to control subjects (e.g., a healthy individual not afflicted with SLE). In another embodiment, the method distinguishes SLE with a sensitivity of at least 80% at a specificity of at least 90% when compared to control subjects. In another embodiment, the method distinguishes SLE with a sensitivity of at least 90% at a specificity of at least 90% when compared to control subjects.

### Antibodies, samples and immunoassays

Antibodies, or immunoglobulins, comprise two heavy chains linked together by disulfide bonds and two light chains, each light chain being linked to a respective heavy chain by disulfide bonds in a "Y" shaped configuration. Each heavy chain has at one end a variable domain (VH) followed by a number of constant domains (CH). Each light chain has a variable domain (VL) at one end and a constant domain (CL) at its other end, the light chain variable domain being aligned with the variable domain of the heavy chain and the light chain constant domain being aligned with the first constant domain of the heavy chain (CH1). The variable domains of each pair of light and heavy chains form the antigen binding site.

The isotype of the heavy chain (gamma, alpha, delta, epsilon or mu) determines immunoglobulin class (IgG, IgA, IgD, IgE or IgM, respectively). The light chain is either of two isotypes (kappa, κ or lambda, λ) found in all antibody classes.

It should be understood that when the terms "antibody" or "antibodies" are used, this is intended to include intact antibodies, such as polyclonal antibodies or monoclonal antibodies (mAbs), as well as proteolytic fragments thereof such as the Fab or F(ab')₂ fragments. Further included within the scope of the invention (for example as immunoassay reagents, as detailed herein) are chimeric antibodies; recombinant and engineered antibodies, and fragments thereof.

Exemplary functional antibody fragments comprising whole or essentially whole variable regions of both light and heavy chains are defined as follows:
(i) Fv, defined as a genetically engineered fragment consisting of the variable region of the light chain and the variable region of the heavy chain expressed as two chains;
(ii) single-chain Fv ("scFv"), a genetically engineered single-chain molecule including the variable region of the light chain and the variable region of the heavy chain, linked by a suitable polypeptide linker.
(iii) Fab, a fragment of an antibody molecule containing a monovalent antigen-binding portion of an antibody molecule, obtained by treating whole antibody with the enzyme papain to yield the intact light chain and the Fd fragment of the heavy chain, which consists of the variable and CH1 domains thereof;
(iv) Fab', a fragment of an antibody molecule containing a monovalent antigen-binding portion of an antibody molecule, obtained by treating whole antibody with the enzyme pepsin, followed by reduction (two Fab' fragments are obtained per antibody molecule); and
(v) F(ab')2, a fragment of an antibody molecule containing a monovalent antigen-binding portion of an antibody molecule, obtained by treating whole antibody with the enzyme pepsin (i.e., a dimer of Fab' fragments held together by two disulfide bonds).

The term "antigen" as used herein is a molecule or a portion of a molecule capable of being bound by an antibody. The antigen is typically capable of inducing an animal to produce antibody capable of binding to an epitope of that antigen. An antigen may have one or more epitopes. The specific reaction referred to above is meant to indicate that the antigen will react, in a highly selective manner, with its corresponding antibody and not with the multitude of other antibodies which may be evoked by other antigens. An "antigenic peptide" is a peptide which is capable of specifically binding an antibody.

In another embodiment, detection of the capacity of an antibody to specifically bind an antigen probe may be performed by quantifying specific antigen-antibody complex formation. The term "specifically bind" as used herein means that the binding of an antibody to an antigen probe is not competitively inhibited by the presence of non-related molecules.

In certain embodiments, the method of the present invention is performed by determining the capacity of an antigen of the invention to specifically bind antibodies of the IgG isotype, or, in other embodiments, antibodies of the IgM, isolated from a subject.

Methods for obtaining suitable antibody-containing biological samples from a subject are well within the ability of those of skill in the art. Typically, suitable samples comprise whole blood and products derived therefrom, such as plasma and serum. In other embodiments, other antibody-containing samples may be used, e.g. CSF, urine and saliva samples.

Numerous well known fluid collection methods can be utilized to collect the biological sample from the subject in order to perform the methods of the invention.

In accordance with the present invention, any suitable immunoassay can be used with the subject peptides. Such techniques are well known to the ordinarily skilled artisan and have been described in many standard immunology manuals and texts. In certain preferable embodiments, determining the capacity of the antibodies to specifically bind the antigen probes is performed using an antigen probe array-based method. Preferably, the array is incubated with suitably diluted serum of the subject (e.g. diluted 1:10) so as to allow specific binding between antibodies contained in the serum and the immobilized antigen probes, washing out unbound serum from the array, incubating the washed array with a detectable label-conjugated ligand of antibodies of the desired isotype, washing out unbound label from the array, and measuring levels of the label bound to each antigen probe.

According to some aspects the methods of the present invention may be practiced using antigen arrays as disclosed in WO 02/08755 and U.S. 2005/0260770 to some of the inventors of the present invention. WO 02/08755 is directed to a system and an article of manufacture for clustering and thereby identifying predefined antigens reactive with undetermined immunoglobulins of sera derived from patient subjects in need of diagnosis of disease or monitoring of treatment. Further disclosed are diagnostic methods, and systems useful in these methods, employing the step of clustering a subset of antigens of a plurality of antigens, said subset of antigens being reactive with a plurality of antibodies being derived from a plurality of patients, and associating or disassociating the antibodies of a subject with the resulting cluster. U.S. Pat. App. Pub. No. 2005/0260770 to some of the inventors of the present invention discloses an antigen array system and diagnostic uses thereof. The application provides a method of diagnosing an immune disease, particularly diabetes type 1, or a predisposition thereto in a subject, comprising determining a capacity of immunoglobulins of the subject to specifically bind each antigen probe of an antigen probe set. The teachings of said disclosures are incorporated in their entirety as if fully set forth herein.

In other embodiments, various other immunoassays may be used, including, without limitation, enzyme-linked immunosorbent assay (ELISA), flow cytometry with multiplex beads (such as the system made by Luminex), surface plasmon resonance (SPR), elipsometry, and various other immunoassays which employ, for example, laser scanning, light detecting, photon detecting via a photo-multiplier, photographing with a digital camera based system or video system, radiation counting, fluorescence detecting, electronic, magnetic detecting and any other system that allows quantitative measurement of antigen-antibody binding.

Various methods have been developed for preparing arrays suitable for the methods of the present invention. State-of-the-art methods involves using a robotic apparatus to apply or "spot" distinct solutions containing antigen probes to closely spaced specific addressable locations on the surface of a planar support, typically a glass support, such as a microscope slide, which is subsequently processed by suitable thermal and/or chemical treatment to attach antigen probes to the surface of the support. Conveniently, the glass surface is first activated by a chemical treatment that leaves a layer of reactive groups such as epoxy groups on the surface, which bind covalently any molecule containing free amine or thiol groups. Suitable supports may also include silicon, nitrocellulose, paper, cellulosic supports and the like.

Preferably, each antigen probe, or distinct subset of antigen probes of the present invention, which is attached to a specific addressable location of the array is attached independently to at least two, more preferably to at least three separate specific addressable locations of the array in order to enable generation of statistically robust data.

In addition to antigen probes of the invention, the array may advantageously include control antigen probes or other standard chemicals. Such control antigen probes may include normalization control probes. The signals obtained from the normalization control probes provide a control for variations in binding conditions, label intensity, "reading" efficiency and other factors that may cause the signal of a given binding antibody-probe ligand interaction to vary. For example, signals, such as fluorescence intensity, read from all other antigen probes of the antigen probe array are divided by the signal (e.g., fluorescence intensity) from the normalization control probes thereby normalizing the measurements. Normalization control probes can be bound to various addressable locations on the antigen probe array to control for spatial variation in antibody-ligand probe efficiency. Preferably, normalization control probes are located at the corners or edges of the array to control for edge effects, as well as in the middle of the array.

The labeled antibody ligands may be of any of various suitable types of antibody ligand. Preferably, the antibody ligand is an antibody which is capable of specifically binding the Fc portion of the antibodies of the subject used. For example, where the antibodies of the subject are of the IgM isotype, the antibody ligand is preferably an antibody capable of specifically binding to the Fc region of IgM antibodies of the subject.

The ligand of the antibodies of the subject may be conjugated to any of various types of detectable labels. Preferably the label is a fluorophore, most preferably Cy3. Alternately, the fluorophore may be any of various fluorophores, including Cy5, fluorescein isothiocyanate (FITC), phycoerythrin (PE), rhodamine, Texas red, and the like. Suitable fluorophore-conjugated antibodies specific for antibodies of a specific isotype are widely available from commercial suppliers and methods of their production are well established.

Antibodies of the subject may be isolated for analysis of their antigen probe binding capacity in any of various ways, depending on the application and purpose. While the subject's antibodies may be suitably and conveniently in the form of blood serum or plasma or a dilution thereof (e.g. 1:10 dilution), the antibodies may be subjected to any desired degree of purification prior to being tested for their capacity to specifically bind antigen probes. The method of the present invention may be practiced using whole antibodies of the subject, or antibody fragments of the subject which comprises an antibody variable region.

### Data analysis

In some embodiments, the methods of the invention may employ the use of learning and pattern recognition analyzers, clustering algorithms and the like, in order to discriminate between reactivity patterns of subjects having a subtype of SLE to control subjects. For example, the methods may include determining the reactivity of antibodies in a test sample to a plurality of antigens, and comparing the resulting pattern to the reactivity patterns of negative and positive control samples using such algorithms and/or analyzers.

Thus, in another embodiment, a significant difference between the reactivity pattern of a test sample compared to a reactivity pattern of a control sample, wherein the difference is computed using a learning and pattern recognition algorithm, indicates that the subject is afflicted with SLE. For example, the algorithm may include, without limitation, supervised or non-supervised classifiers including statistical algorithms including, but not limited to, principal component analysis (PCA), partial least squares (PLS), multiple linear regression (MLR), principal component regression (PCR), discriminant function analysis (DFA) including linear discriminant analysis (LDA), and cluster analysis including nearest neighbor, artificial neural networks, coupled two-way clustering algorithms, multi-layer perceptrons (MLP), generalized regression neural network (GRNN), fuzzy inference systems (FIS), self-organizing map (SOM), genetic algorithms (GAS), neuro-fuzzy systems (NFS), adaptive resonance theory (ART).

In certain embodiments, one or more algorithms or computer programs may be used for comparing the amount of each antibody quantified in the test sample against a predetermined cutoff (or against a number of predetermined cutoffs). Alternatively, one or more instructions for manually performing the necessary steps by a human can be provided.

Algorithms for determining and comparing pattern analysis include, but are not limited to, principal component analysis, Fischer linear analysis, neural network algorithms, genetic algorithms, fuzzy logic pattern recognition, and the like. After analysis is completed, the resulting information can, for example, be displayed on display, transmitted to a host computer, or stored on a storage device for subsequent retrieval.

Many of the algorithms are neural network based algorithms. A neural network has an input layer, processing layers and an output layer. The information in a neural network is distributed throughout the processing layers. The processing layers are made up of nodes that simulate the neurons by the interconnection to their nodes. Similar to statistical analysis revealing underlying patterns in a collection of data, neural networks locate consistent patterns in a collection of data, based on predetermined criteria.

Suitable pattern recognition algorithms include, but are not limited to, principal component analysis (PCA), Fisher linear discriminant analysis (FLDA), soft independent modeling of class analogy (SIMCA), K-nearest neighbors (KNN), neural networks, genetic algorithms, fuzzy logic, and other pattern recognition algorithms. In some embodiments, the Fisher linear discriminant analysis (FLDA) and canonical discriminant analysis (CDA) as well as combinations thereof are used to compare the output signature and the available data from the database.

In other embodiments, principal component analysis is used. Principal component analysis (PCA) involves a mathematical technique that transforms a number of correlated variables into a smaller number of uncorrelated variables. The smaller number of uncorrelated variables is known as principal components. The first principal component or eigenvector accounts for as much of the variability in the data as possible, and each succeeding component accounts for as much of the remaining variability as possible. The main objective of PCA is to reduce the dimensionality of the data set and to identify new underlying variables.

Principal component analysis compares the structure of two or more covariance matrices in a hierarchical fashion. For instance, one matrix might be identical to another except that each element of the matrix is multiplied by a single constant. The matrices are thus proportional to one another. More particularly, the matrices share identical eigenvectors (or principal components), but their eigenvalues differ by a constant. Another relationship between matrices is that they share principal components in common, but their eigenvalues differ. The mathematical technique used in principal component analysis is called eigenanalysis. The eigenvector associated with the largest eigenvalue has the same direction as the first principal component. The eigenvector associated with the second largest eigenvalue determines the direction of the second principal component. The sum of the eigenvalues equals the trace of the square matrix and the maximum number of eigenvectors equals the number of rows of this matrix.

In another embodiment, the algorithm is a classifier. One type of classifier is created by "training" the algorithm with data from the training set and whose performance is evaluated with the test set data. Examples of classifiers used in conjunction with the invention are discriminant analysis, decision tree analysis, receiver operator curves or split and score analysis.

The term "decision tree" refers to a classifier with a flow-chart-like tree structure employed for classification. Decision trees consist of repeated splits of a data set into subsets. Each split consists of a simple rule applied to one variable, e.g., "if value of "variable 1" larger than "threshold 1"; then go left, else go right". Accordingly, the given feature space is partitioned into a set of rectangles with each rectangle assigned to one class.

The terms "test set" or "unknown" or "validation set" refer to a subset of the entire available data set consisting of those entries not included in the training set. Test data is applied to evaluate classifier performance.

The terms "training set" or "known set" or "reference set" refer to a subset of the respective entire available data set. This subset is typically randomly selected, and is solely used for the purpose of classifier construction.

### Diagnosing SLE

The methods of the invention are useful in diagnosing systemic lupus erythematosus (SLE). In some embodiments, SLE is cutaneous SLE. In additional embodiments, said SLE is subacute cutaneous SLE or SCLE. Other forms of lupus may also be diagnosed using the methods and kits of the invention, such as nephritis, extrarenal, cerebritis, pediatric, non-renal, discoid (DLE), and alopecia. Each possibility is a separate embodiment of the invention. "Lupus" as used herein is an autoimmune disease or disorder involving antibodies that attack connective tissue.

As used herein the term "diagnosing" or "diagnosis" refers to the process of identifying a medical condition or disease (e.g., SLE) by its signs, symptoms, and in particular from the results of various diagnostic procedures, including e.g. detecting the reactivity of antibodies in a biological sample (e.g. serum) obtained from an individual, to a plurality of antigens. Furthermore, as used herein the term "diagnosing" or "diagnosis" encompasses screening for a disease, detecting a presence or a severity of a disease, distinguishing a disease from other diseases including those diseases that may feature one or more similar or identical symptoms, providing prognosis of a disease, monitoring disease progression or relapse, as well as assessment of treatment efficacy and/or relapse of a disease, disorder or condition, as well as selecting a therapy and/or a treatment for a disease, optimization of a given therapy for a disease, monitoring the treatment of a disease, and/or predicting the suitability of a therapy for specific patients or subpopulations or determining the appropriate dosing of a therapeutic product in patients or subpopulations.

As demonstrated herein below (Example 4), a sample was obtained from a subject when she was in a healthy state and was negative for standard anti-DNA antibodies, but 8 months later she began suffering from non-specific symptoms and was eventually found, after 7 more months, to fulfill the criteria for a diagnosis of SLE. Thus the antigen-microarray signature might also detect pre-clinical SLE. In a particular embodiment, the method and kits of the invention are useful for diagnosing pre-clinical SLE. In yet another particular embodiment, the method and kits of the invention are useful for early detection of SLE.

In one embodiment, the subject being diagnosed according to the methods of the invention is symptomatic. In other embodiments, the subject is asymptomatic.

The diagnostic procedure can be performed in vivo or in vitro, preferably in vitro.

### Criteria for diagnosing SLE

The 1982 American College of Rheumatology (ACR) criteria summarize features necessary to diagnose SLE. The presence of 4 of the 11 criteria yields a sensitivity of 85% and a specificity of 95% for SLE. Patients with SLE may present with any combination of clinical features and serologic evidence of lupus.
- Serositis - Pleurisy, pericarditis on examination or diagnostic ECG or imaging
- Oral ulcers - Oral or nasopharyngeal, usually painless; palate is most specific
- Arthritis - Nonerosive, two or more peripheral joints with tenderness or swelling
- Photosensitivity - Unusual skin reaction to light exposure
- Blood disorders - Leukopenia (<4 X 10³ cells/µL on more than one occasion), lymphopenia (<1500 cells/µL on more than one occasion), thrombocytopenia (<100 X 10³ cells/µL in the absence of offending medications), hemolytic anemia
- Renal involvement - Proteinuria (>0.5 g/d or 3+ positive on dipstick testing) or cellular casts
- ANAs - Higher titers generally more specific (>1:160); must be in the absence of medications associated with drug-induced lupus
- Immunologic phenomena - dsDNA; anti-Smith (Sm) antibodies; antiphospholipid antibodies (anticardiolipin immunoglobulin G [IgG] or immunoglobulin M [IgM] or lupus anticoagulant); biologic false-positive serologic test results for syphilis, lupus erythematosus (LE) cells (omitted in 1997)
- Neurologic disorder - Seizures or psychosis in the absence of other causes
- Malar rash - Fixed erythema over the cheeks and nasal bridge, flat or raised
- Discoid rash - Erythematous raised-rimmed lesions with keratotic scaling and follicular plugging, often scarring

Two of the most commonly used instruments for SLE diagnosis are the Systemic Lupus Erythematosus Disease Activity Index (SLEDAI) and the Systemic Lupus Activity Measure (SLAM).

### SLE Disease Activity Index (SLEDAI)

The SLEDAI is an index that measures disease activity by weighting the importance of each organ system involved. The SLEDAI includes 24 items, representing nine organ systems. The variables are obtained by history, physical examination and laboratory assessment. Each item is weighted from 1 to 8 based on the significance of the organ involved. For example, mouth ulcers are scored as 2, while seizures are scored as 8. The laboratory parameters that are included in the SLEDAI include white blood cell count, platelet count, urinalysis, serum C3, C4 and anti-dsDNA. The total maximum score is 105.

### Systemic Lupus Activity Measure (SLAM)

The SLAM includes 32 items representing 11 organ systems. The items are scored not only as present/absent, but graded on a scale of 1 to 3 based on severity. The total possible score for the SLAM is 86. Both the SLEDAI and the SLAM have been shown to be valid, reliable, and sensitive to change over time (Liang et al. 1989, Arth Rheum 32:1107-18), and are widely used in research protocols and clinical trials. These indices are particularly useful for examining the value of newly proposed serologic or inflammatory markers of disease activity in SLE.

Despite the obvious utility of these instruments, there are some drawbacks. First, there is not always complete agreement between the SLAM and the SLEDAI in the same set of patients. There are several possible reasons for these discrepancies. Unlike the SLEDAI, the SLAM includes constitutional symptoms such as fatigue and fever, which may or may not be considered attributable to active SLE; this activity index relies on physician interpretation. In addition, the SLEDAI does not capture mild degrees of activity in some organ systems and does not have descriptors for several types of activity, such as hemolytic anemia.

The following examples are presented in order to more fully illustrate some embodiments of the invention. They should, in no way be construed, however, as limiting the broad scope of the invention.

### EXAMPLES

### Materials and Methods

### Human Subjects

The study was approved by the institutional review board of each participating clinical unit; informed consent was obtained from all participants.

Three groups of SLE patients and a control group were studied: 15 patients in renal remission; 14 patients with active lupus nephritis; 11 patients without renal involvement; and 16 age-matched and sex-matched healthy controls. Blood samples and clinical data were collected from SLE patients arriving at the Rheumatology Unit and Hematology Department of the Sheba Medical Center, Israel; the Rheumatology Unit at the Hadassah Medical Center, Ein Kerem, Jerusalem, Israel; and the Cellular Biology and Immunogenetics Unit at the Corporacion para Investigaciones Biologicas, Medellin, Colombia. All patients fulfilled the American College of Rheumatology criteria for SLE (Tan et al: Arthritis. Rheum. 25:1271, 1982; updated by MC Hochberg, Arthritis. Rheum. 1997; 40:1725.). SLE patients with active lupus nephritis were defined by a SLEDAI of ≥ 8 and one of the following: new onset proteinuria of ≥ 1g; an increase in the urinary protein:creatinine ratio ≥ 2; or an increase of ≥ 50% of the baseline serum creatinine. SLE patients in renal remission were individuals who were once diagnosed as having active lupus nephritis as defined above, but were now with a systemic lupus erythematosus disease activity index (SLEDAI) ≤ 4 and one of the following: a return to baseline serum creatinine with a decrease in proteinuria to within 25% of their baseline level, or a return to baseline proteinuria and a return of serum creatinine to within 25% of their baseline level. All patients in remission remained stable for at least 6 months; the mean time in remission was 8 years; the range was 3 months to 30 years. Patients without known renal involvement were known not to have suffered from kidney involvement in the past and during a follow up of at least 1 year. The mean time from diagnosis was 7 years; the range was from 0.5 to 27 years. Additional patient data are shown in Table 2 (values are presented as mean ± standard error or percent).

**Table 2- The clinical characteristics of the SLE patients**

| **Characteristic** | **Renal Involvement** | | |
|---|---|---|---|
| | **None (n=11)** | **Remission (n=15)** | **Active (n=14)** |
| **Sex (% Female)** | 100% | 87% | 86% |
| **Age (years)** | 40.6±4.4 | 36.1±3.2 | 32.4±2.5 |
| **SLEDAI** | 1.8±1 | 1.1±0.4 | 14.6±2.2 |
| **Anti DNA Ab (%positive)** | 18% | 40% | 86% |
| **Complement (%decreased)** | 9% | 7% | 83% |
| **Creatinine mg/dL** | 0.75±0.03 | 0.97±0.08 | 1.2±0.26 |
| **Duration of disease (months)** | 84±29 | 150±23 | 118±25 |

### Antigen Microarrays and Serum Testing

Antigen microarray chips were prepared as described in Merbl et al. 2007 J. Clin. Invest. 117:712-8 and Quintana et al. 2006, Lupus 15:428-30; and Quintana et al. 2004, Proc. Natl. Acad. Sci. USA. 101: 14615-21.

694 antigens, each at its optimal concentration (mostly at 1 mg/ml), were spotted in triplicate on Epoxy-activated glass substrates using a 48-pin robot (Microgrid 600, Genomics Solutions, Ann Arbor, MI, USA). These antigens included proteins, synthetic peptides from the sequences of selected proteins, nucleotides, phospholipids, and other self and non-self molecules. The microarrays were then blocked for 1 hour at 37°C with 1% bovine serum albumin. Test serum in blocking buffer (1:10 dilution) was incubated under a coverslip for 1 hour at 37°C. The arrays were then washed and incubated for 1 hour at 37°C with a 1:500 dilution of two detection antibodies, mixed together: a goat anti-human IgG Cy3-conjugated antibody, and a goat anti-human IgM Cy5-conjugated antibody, (both purchased from Jackson ImmunoResearch Laboratories Inc. West Grove, PA, USA). Image acquisition was performed by laser (Agilent Technologies, Santa Clara, CA, USA) and the results were analyzed by Quantarray software (Packard BioChip Technologies, Billerica, MA, USA) and by additionally developed software. The quantitative range of signal intensity of binding to each antigen spot was 0-65,000; this range of detection made it possible to obtain reliable data at the 1:10 dilution of test samples (Quintana et al.. Proc. Natl. Acad. Sci. USA. 105:18889-94). Anti-DNA antibodies were also measured separately using ELISA (QUANTA-Lite, Inova, San Diego, CA) and the Farr assay (Wold et al., Science 161: 806).

### Image Analysis and Data Processing

Technically faulty spots were manually excluded by visual inspection of each slide. The foreground and background intensities of multiple spots of each antigen were then averaged, and a log-base-10 value of the difference between the foreground and the background was calculated; differences less than 500 were clamped to 500 and then log transformed. To control for differences between different slides, the average laser intensity value of each slide (in the corresponding IgM or IgG channel) was then subtracted. The value of each antigen was then shifted such that its minimal value over the entire dataset equals zero. The resulting value was taken as the *antigen reactivity* of the antibodies binding to that spotted antigen. Antigens that showed zero reactivity in more than 80% of the slides were excluded, as were antigens whose coefficient of variation across slides was lower than 20%. In this way, the 694 IgM and the 694 IgG antigen reactivities were reduced to about 930 reactivities, almost evenly split between IgM and IgG channels.

### Statistical Analysis

Statistical analysis was done to identify antigen reactivities and groups of antigen reactivities that could distinguish between the different groups of subjects. The different comparisons were performed in the same manner, designated generically here as groups A and B, each consisting of n_{A} and n_{B} subjects (each subject being a separate microarray slide). To estimate the quality of the differentiation between groups A and B, a leave-one-out (LOO) procedure was applied (Duda et al., 2001, Pattern Classification. John Wiley and Sons, Inc., New York. 654 pp): One subject out of n_{A}+n_{B} was left aside, and the rest of the n_{A}+n_{B}-1 subjects were used to select candidate antigens for separating groups A and B. A T-test between groups A and B (excluding the one subject) was applied, and the d antigens that passed a false discovery rate (FDR) threshold of 5% were selected (Benjamini et al., 1995, Journal of the Royal Statistical Society B. 57:289-300). These antigens were then used to classify the left-out subject using the K-nearest-neighbors algorithm (Duda et al., 2001), with K=3. More specifically, the left-out subject seeks its 3 nearest subject data points in the d-dimensional space containing the other n_{A}+n_{B}-1 subjects, and performs its classification according to the majority class of these 3 subjects. This procedure was repeated for all n_{A}+n_{B} subjects; the performance, which appears in Table 3 was simply the number of misclassifications, quantified as specificity (1-false positive rate) and sensitivity (1-false negative rate) measures.

The composition of the list of separating antigen reactivities can vary depending on the specific subject that is left out in the particular LOO cross-validation run. To further identify antigen reactivities that play an important role in separating groups A and B, the antigen reactivities that appeared in the candidate lists in at least 90% of the LOO tests was selected. The LOO procedure was repeated with each of these selected antigen reactivities, acting alone, by classifying the left-out subject using a simple threshold criterion: the reactivity values of the n_{A}+n_{B}-1 training subjects that threshold value which maximized the specificity was found, and then classified the left-out point using this threshold. The average LOO specificity and sensitivity of this classification over the n_{A}+n_{B} subjects appear for each of the selected antigens in Tables 4 and 6. To test the performance of combinations of antigen reactivities, the particular combination was projected onto a one-dimensional space using principal component analysis (PCA), and the combination was treated as with the above single reactivities.

In addition to the LOO test, the subjects in groups A and B, were taken together with their list of differentiating antigen reactivities, and used to classify a test set C via three nearest neighbors. For example, the entire list of frequent antigen reactivities that separated healthy controls from SLE subjects in renal remission was used to classify the other two SLE groups - those in renal relapse and those without renal involvement. Figure 2 displays a projection of these antigen reactivities onto a 3-dimensional space using a principal component analysis (PCA) representation (Duda et al., 2001).

### EXAMPLE 1

### Antigen microarray reactivities differentiate SLE subjects from healthy controls

Table 3 shows a global analysis of the 930 total antibody reactivities of the healthy control subjects compared with those of three SLE groups based on a leave one out (LOO) test: all SLE subjects; SLE subjects in renal remission; and SLE subjects with active lupus nephritis. The average LOO sensitivity and specificity are presented for a K=3 nearest neighbor classifier.

The analysis shows that the microarray reactivities clearly separated the three groups of SLE subjects from the healthy controls. A comparison between healthy controls and SLE subjects without renal involvement does not appear in the table because no antigen exceeded an FDR level of 5% in some of the LOO tests. Moreover, the various sub-groups of SLE subjects, with the limited numbers available for testing, also could not be separated from one another because none of the antigen reactivities passed an FDR level of 5% in any of the LOO tests..

**Table 3- Performance of the antigen microarray on different classification tasks**

| **Healthy controls compared to:** | **Sensitivity** | **Specificity** |
|---|---|---|
| All SLE subjects | 90% | 81% |
| SLE subjects in renal remission | 93% | 100% |
| SLE patients with active lupus nephritis | 86% | 100% |

### EXAMPLE 2

### SLE subjects manifest both up-regulation and down-regulation of individual reactivities

Table 5 lists the particular antibody reactivities that distinguished all the SLE patients as a group from the healthy control subjects. Eight IgG antibody reactivities were up-regulated in the SLE group as compared to control: dsDNA, ssDNA, HyaluronicAcid, EBV, BMP4, F50, HGF and hsp60p18. Further, thirteen IgM antibody reactivities were down-regulated in the SLE group as compared to control: MPO, IGFBP1, CD99, Cardiolipin, actin, CMV, Collagen-III, Collagen-IV, RV, S100A4, CITED1 and FHIT.

Table 4 lists the particular antibody reactivities that distinguished all the SLE patients as a group from the healthy control subjects. Figure 1 shows the relative amounts of antibody reactivities to the antigens listed in Table 4 in each serum. The differences between the two groups for each of these antigens exceeded an FDR level of 5% (p<0.0007). Four IgG antibody reactivities were up-regulated in the SLE group: classic reactivities to dsDNA and ssDNA, reactivity to Epstein-Barr Virus (EBV), which has been previously found to be strongly associated with SLE (Barzilai et al., 2007, Ann. N Y Acad. Sci. 1108:567-77), and a surprising reactivity to hyaluronic acid (Figure 1).

**Table 4- Antibody reactivities that individually distinguish the healthy control subjects from all the SLE patients**

| **Antigen** | **Immunoglubulin Isotype** | **Sensitivity (%)** | **Specificity (%)** |
|---|---|---|---|
| **SLE Up-regulated** | | | |
| dsDNA | IgG | 58 | 87 |
| ssDNA | IgG | 75 | 94 |
| HyaluronicAcid | IgG | 86 | 86 |
| EBV | IgG | 70 | 88 |
| **SLE Down-regulated** | | | |
| MPO | IgM | 78 | 88 |
| IGFBP1 | IgM | 59 | 82 |
| CD99 | IgM | 61 | 93 |
| Cardiolipin | IgM | 60 | 81 |
| **All eight antigens** | IgM + IgG | 93 | 88 |
| **All four IgG antigens** | IgG | 90 | 88 |
| **All four IgM antigens** | IgM | 68 | 88 |

Four novel antigen reactivities, all IgM, were found to be down-regulated in SLE: Insulin-like growth factor binding protein 1 (IGFBP1), CD99, Cardiolipin and myeloperoxidase (MPO). The IgM antibody reactivities of SLE subjects to these antigens tended to be low or undetectable compared with the healthy controls (Figure 1). In contrast to the decreased IgM reactivities to MPO and to cardiolipin, increased IgG antibodies to these antigens have been associated with SLE and other vasculitis-related diseases (Barzilai et al., 2007; Sen and Isenberg, 2003 Lupus 12:651-8; Love, P.E. and Santoro, S.A. 1990, Ann. Intern. Med. 112:682-98; Moreland et al., 1991. Clin. Immunol. Immunopathol. 60:412-8).

Table 4 shows that, except for IgG reactivity to hyaluronic acid, the other individual reactivities showed sensitivity for SLE of < 80%. However, the specificities of each reactivity, whether increased IgG or decreased IgM, were > 80%. The combination of all eight reactivites increased the sensitivity to > 90%; the combination of four IgG increased rectivities was more sensitive than the combination of the four IgM decreased reactivities: 90% compared with 68%, respectively. The specificites of each of the combined sets were equal at 88%.

**Table 5- Antibody reactivities that individually distinguish the healthy control subjects from all the SLE patients**

| **Antigen** | **Immunoglubulin Isotype** | **Sensitivity** (%) | **Specificity** (%) |
|---|---|---|---|
| **SLE Up-regulated** | | | |
| dsDNA | IgG | 58 | 87 |
| ssDNA | IgG | 75 | 94 |
| HyaluronicAcid | IgG | 86 | 86 |
| EBV | IgG | 70 | 88 |
| BMP4 | IgG | 38 | 88 |
| F50 | IgG | 38 | 88 |
| HGF | IgG | 69 | 82 |
| hsp60p18 | IgG | 49 | 87 |
| **SLE Down-regulated** | | | |
| MPO | IgM | 78 | 88 |
| IGFBP1 | IgM | 59 | 82 |
| CD99 | IgM | 61 | 93 |
| Cardiolipin | IgM | 60 | 81 |
| actin | IgM | 58 | 77 |
| CMV | IgM | 53 | 87 |
| CollagenIII | IgM | 64 | 83 |
| CollagenIV | IgM | 57 | 77 |
| HRP | IgM | 44 | 88 |
| RV | IgM | 38 | 88 |
| S100A4 | IgM | 36 | 88 |
| CITED 1 | IgM | 45 | 88 |
| FHIT | IgM | 23 | 88 |

### EXAMPLE 3

### SLE subjects in remission maintain an SLE signature

An important question is whether clinical renal remission is associated with a return of the SLE antibody pattern to a healthy state. Table 6 shows that SLE patients in clinical remission still maintained an SLE signature. These patients manifested significantly up-regulated IgG reactivities to the same four antigens that characterized the general set of SLE subjects: dsDNA, ssDNA, hyaluronic acid and EBV. Moreover, those in remission manifested down-regulation of three IgM reactivities, of which two were characteristic of the SLE group as a whole: decreased IgM reactivity to CD99 and to MPO were present in both groups, but those in remission manifested decreased IgM reactivity to collagen III rather than to cardiolipin and to IGFBP1 (Figure 1).

Table 6 also shows that combining the four increased IgG snd thr three decreased IgM reactivities led to 100% sensitivity and 94% specificity. Thus, a combination of reactivities may provide a higher degree of accuracy than any of the component reaction alone. Note too that the set of combined decreased IgM reactivities performed as well as did the set of combined increase IgM reactivities appears to be a characteristic of SLE.

**Table 6- Antibody reactivities that individually distinguish the healthy control subjects from the SLE patients in renal remssion**

| **Antigen** | **Ig Isotype** | **Sensitivity** (%) | **Specificity** (%) |
|---|---|---|---|
| **SLE Up-regulated** | | | |
| ssDNA | IgG | 87 | 94 |
| dsDNA | IgG | 47 | 87 |
| HyaluronicAcid | IgG | 93 | 86 |
| EBV | IgG | 73 | 88 |

| **SLE Down-regulated** | | | |
|---|---|---|---|
| MPO | IgM | 87 | 94 |
| Collagen III | IgM | 73 | 83 |
| CD99 | IgM | 77 | 93 |
| All seven antigens | IgM + IgG | 100 | 94 |
| All four IgG antigens | IgG | 93 | 94 |
| All three IgM antigens | IgM | 93 | 94 |

### EXAMPLE 4

### The SLE remission signature also characterizes other SLE groups

To determine whether the list of antigen reactivities characteristic of SLE in remission might be applicable to SLE generally, we used the seven antigens that separated subjects in remission from healthy controls (Table 6) to classify the 14 SLE patients with active lupus nephritis and the 11 SLE patients without renal involvement.

These 25 SLE patients were classified via a three nearest neighbours algorithm, based on 15 SLE patients in remission and 16 healthy controls. Twenty-three of these 25 SLE subjects were correctly classifies, generating a sensitivity of 92%. Only 2 out of the 26 SLE patients were misclassified (not shown).

Figure 2 displays a 3-dimensional PCA representation (projected from the space spanned by the 7 separating antigens) of healthy control subjects and those with various sub-groups of SLE. The healthy controls were clearly separated by the seven antigen reactivities from the SLE subjects in remission. Moreover, the individuals in long-term remission, in acute lupus nephritis, or without renal involvement were completely overlapping. In other words, the remission list of antigen reactivities in Table 3 constitutes an SLE antibody signature that includes SLE subjects with active lupus nephritis and those without renal involvement. Remarkably, the subject marked by a blue star, who was projected into the SLE domain of Figure 2; serum from this subject was obtained when she was in a healthy state and was negative for standard anti-DNA antibodies, but 8 months later she began suffering from non-specific symptoms and was eventually found, after 7 more months, to fulfill the criteria for a diagnosis of SLE. Thus the antigen-microarray signature also detects pre-clinical SLE.

### References

Sherer, Y., Gorstein, A., Fritzler, M.J., Shoenfeld, Y. 2004. Autoantibody explosion in systemic lupus erythematosus: more than 100 different antibodies found in SLE patients. Semin. Arthritis. Rheum. 34:501-37.
Criteria published by EM Tan et al: Arthritis. Rheum. 25:1271, 1982; updated by MC Hochberg, Arthritis. Rheum. 1997; 40:1725.
Quintana, F.J., Farez, M.F., Viglietta, V., Iglesias, A.H., Merbl, Y., Izquierdo, G., Lucas, M., Basso, A.S., Khoury, S.J., Lucchinetti, C.F., Cohen, I.R., Weiner, H.L. 2008. Antigen microarrays identify unique serum autoantibody signatures associated with different clinical forms and pathologic subtypes of multiple sclerosis. Proc. Natl. Acad. Sci. U S A. 105:18889-94.
Merbl, Y., Zucker-Toledano, M., Quintana, F.J., Cohen, I.R. 2007. Newborn humans manifest autoantibodies to defined self molecules detected by antigen microarray informatics. J. Clin. Invest. 117:712-8.
Barzilai, O., Sherer, Y., Ram, M., Izhaky, D., Anaya, J.M., Shoenfeld, Y. 2007 Epstein-Barr virus and cytomegalovirus in autoimmune diseases: are they truly notorious? A preliminary report. Ann. N Y Acad. Sci. 1108:567-77.
Manolova, I., Dancheva, M., Halacheva, K. 2002. Predominance of IgG1 and IgG3 subclasses of autoantibodies to neutrophil cytoplasmic antigens in patients with systemic lupus erythematosus. Rheumatol. Int. 21:227-33.
Sen, D., Isenberg, D.A. 2003. Antineutrophil cytoplasmic autoantibodies in systemic lupus erythematosus. Lupus 12:651-8.
Love, P.E., Santoro, S.A. 1990. Antiphospholipid antibodies: anticardiolipin and the lupus anticoagulant in systemic lupus erythematosus (SLE) and in non-SLE disorders. Prevalence and clinical significance. Ann. Intern. Med. 112:682-98.
Moreland, L.W., Gay, R.E., Gay, S. 1991. Collagen autoantibodies in patients with vasculitis and systemic lupus erythematosus. Clin. Immunol. Immunopathol. 60:412-8.
Isenberg, D.A., Colaco, C.B., Dudeney, C., Todd-Pokropek, A., Snaith, M.L. 1986. The relationship of anti-DNA antibody idiotypes and anti-cardiolipin antibodies to disease activity in systemic lupus erythematosus. Medicine 65:46-55.
Li, Q.Z., Zhou, J., Wandstrat, A.E., Carr-Johnson, F., Branch, V., Karp, D.R., Mohan, C., Wakeland, E.K., Olsen, N.J. 2007. Protein array autoantibody profiles for insights into systemic lupus erythematosus and incomplete lupus syndromes. Clin. Exp. Immunol. 147:60-70.
Li, Q.Z., Xie, C., Wu, T., Mackay, M., Aranow, C., Putterman, C., Mohan, C. 2005. Identification of autoantibody clusters that best predict lupus disease activity using glomerular proteome arrays. J. Clin. Invest. 115:3428-39.
Quintana, F.J., Merbl, Y., Sahar, E., Domany, E., Cohen, I.R. 2006. Antigen-chip technology for accessing global information about the state of the body. Lupus 15:428-30.
Quintana, F.J., Hagedorn, P.H., Elizur, G., Merbl, Y., Domany, E., Cohen, I.R. 2004. Functional immunomics: microarray analysis of IgG autoantibody repertoires predicts the future response of mice to induced diabetes. Proc. Natl. Acad. Sci. U S A. 101 Suppl 2: 14615-21.
Wold, R.T., Young, F.E., Tan, E.M., Farr, R.S. 1968. Deoxyribonucleic acid antibody: A method to detect its primary interaction with deoxyribonucleic acid. Science 161: 806.
Duda, R.O., Hart, P.E., Stork, D.G. 2001. Pattern Classification. John Wiley and Sons, Inc., New York. 654 pp.
Benjamini, Y., Hochberg, Y. 1995. Controlling the False Discovery Rate: a Practical and Powerful Approach to Multiple Testing. Journal of the Royal Statistical Society B. 57:289-300.

The foregoing description of the specific embodiments will so fully reveal the general nature of the invention that others can, by applying current knowledge, readily modify and/or adapt for various applications such specific embodiments without undue experimentation and without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. The means, materials, and steps for carrying out various disclosed functions may take a variety of alternative forms without departing from the invention.

## Claims

1. A method of diagnosing systemic lupus erythematosus (SLE) in a subject, the method comprising:
(i) determining the reactivity of IgG and IgM antibodies in a sample obtained from the subject to at least four antigens selected from the group consisting of: IGFBP1, CD99, hyaluronic acid, EBV, ssDNA, dsDNA, MPO, cardiolipin, collagen III, collagen IV, actin, BMP4, CMV, F50, HGF, HSP60p18, RV, S100A4, CITED1 and FHIT, thereby determining the reactivity pattern of the sample to the plurality of antigens; and
(ii) comparing the reactivity pattern of said sample to a control reactivity pattern;
wherein an increased reactivity of IgG antibodies to a plurality of antigens selected from the group consisting of: hyaluronic acid, EBV, ssDNA, dsDNA, BMP4, F50, HGF and HSP60p18, and/or a decreased reactivity of IgM antibodies to a plurality of antigens selected from the group consisting of: IGFBP1, CD99, MPO, cardiolipin, collagen III, collagen IV, actin, CMV, RV, S100A4, CITED1 and FHIT compared to the reactivity pattern of a control sample is an indication that the subject is afflicted with SLE.

2. The method of claim 1, wherein the plurality of antigens comprise at least five antigens.

3. The method of claim 1, wherein the plurality of antigens comprise at least six antigens.

4. The method of claim 1, wherein the plurality of antigens comprise at least seven antigens.

5. The method of claim 1, wherein the plurality of antigens comprise at least eight antigens.

6. The method of claim 1, wherein the plurality of antigens consist of IGFBP1, CD99, hyaluronic acid, EBV, ssDNA, dsDNA, MPO, cardiolipin and collagen III.

7. The method of claim 1, comprising determining the reactivity of a plurality of IgG antibodies and a plurality of IgM antibodies in said sample to said plurality of antigens.

8. The method of claim 1, comprising determining the reactivity of IgG antibodies in the sample obtained from the subject to a plurality of antigens selected from the group consisting of: hyaluronic acid, EBV, ssDNA and dsDNA.

9. The method of claim 1, comprising determining the reactivity of IgM antibodies in the sample obtained from the subject to a plurality of antigens selected from the group consisting of: CD99, IGFBP1, MPO, cardiolipin and Collagen III.

10. The method of claim 1 comprising determining the reactivity of IgM antibodies in the sample obtained from the subject to a plurality of antigens selected from the group consisting of: CD99, IGFBP1, MPO and cardiolipin.

11. The method of claim 1, comprising determining the reactivity of IgM antibodies in the sample obtained from the subject to a plurality of antigens selected from the group consisting of: CD99, MPO and Collagen III, wherein a down-regulation between the reactivity pattern of said sample obtained from the subject compared to the reactivity pattern of a control sample is an indication that the subject is afflicted with SLE in renal remission.

12. The method of claim 1, wherein the sample is a serum sample.

13. The method of claim 1, wherein the control is selected from the group consisting of a sample from at least one healthy individual, a panel of control samples from a set of healthy individuals, and a stored set of data from control individuals.

14. The method of claim 1, further comprising diluting the sample 1:10 before determining the reactivity of antibodies in the sample.

15. The method of claim 1, wherein said plurality of antigens is used in the form of an antigen array.

## Patentansprüche

1. Verfahren zur Diagnose von systemischem Lupus Erythematosus (SLE) in einem Individuum, welches umfasst:
(i) Bestimmen der Reaktivität von IgG- und IgM-Antikörpern in einer von dem Individuum erhaltenen Probe gegen mindestens vier Antigene, ausgewählt aus der Gruppe bestehend aus: IGFBP1, CD99, Hyaluronsäure, EBV, ssDNA, dsDNA, MPO, Cardiolipin, Kollagen III, Kollagen IV, Aktin, BMP4, CMV, F50, HGF, HSP60p18, RV, S100A4, CITED1 und FHIT, wobei das Reaktivitätsmuster der Probe gegen die mehreren Antigene bestimmt wird; und
(ii) Vergleichen des Reaktivitätsmusters der Probe gegen ein Kontrol-Reaktivitätsmuster;
wobei eine gesteigerte Reaktivität von IgG-Antikörpern gegen mehrere Antigene ausgewählt aus der Gruppe bestehend aus: Hyaluronsäure, EBV, ssDNA, dsDNA, BMP4, F50, HGF und HSP60p18 und/oder eine verringerte Reaktivität von IgM-Antikörpern gegen mehrere Antigene ausgewählt aus der Gruppe bestehend aus: IGFBP1, CD99, MPO, Cardiolipin, Kollagen III, Kollagen IV, Aktin, CMV, RV, S100A4, CITED1 und FHIT im Vergleich zu dem Reaktivitätsmuster einer Kontroll-Probe ein Anzeichen dafür ist, dass das Individuum an SLE leidet.

2. Verfahren nach Anspruch 1, wobei die mehreren Antigene mindestens fünf Antigene umfassen.

3. Verfahren nach Anspruch 1, wobei die mehreren Antigene mindestens sechs Antigene umfassen.

4. Verfahren nach Anspruch 1, wobei die mehreren Antigene mindestens sieben Antigene umfassen.

5. Verfahren nach Anspruch 1, wobei die mehreren Antigene mindestens acht Antigene umfassen.

6. Verfahren nach Anspruch 1, wobei die mehreren Antigene sind: IGFBP1, CD99, Hyaluronsäure, EBV, ssDNA, dsDNA, MPO, Cardiolipin und Kollagen III.

7. Verfahren nach Anspruch 1, welches umfasst, Bestimmen der Reaktivität mehrerer IgG-Antikörper und mehrerer IgM-Antikörper in der Probe gegen die mehreren Antigene.

8. Verfahren nach Anspruch 1, welches umfasst, Bestimmen der Reaktivität von IgG-Antikörpern in der von dem Individuum erhaltenen Probe gegen mehrere Antigene ausgewählt aus der Gruppe bestehend aus: Hyaluronsäure, EBV, ssDNA und dsDNA.

9. Verfahren nach Anspruch 1, welches umfasst, Bestimmen der Reaktivität von IgM-Antikörpern in der von dem Individuum erhaltenen Probe gegen mehrere Antigene ausgewählt aus der Gruppe bestehend aus: CD99, IGFBP1, MPO, Cardiolipin und Kollagen III.

10. Verfahren nach Anspruch 1, welches umfasst, Bestimmen der Reaktivität von IgM-Antikörpern in der von dem Individuum erhaltenen Probe gegen mehrere Antigene ausgewählt aus der Gruppe bestehend aus: CD99, IGFBP1, MPO und Cardiolipin.

11. Verfahren nach Anspruch 1, welches umfasst, Bestimmen der Reaktivität von IgM-Antikörpern in der von dem Individuum erhaltenen Probe gegen mehrere Antigene ausgewählt aus der Gruppe bestehend aus: CD99, MPO und Kollagen III, wobei ein Herunterregulieren zwischen dem Reaktivitätsmuster der von dem Individuum erhaltenen Probe im Vergleich zu dem Reaktivitätsmuster einer Kontrollprobe ein Anzeichen dafür ist, dass das Individuum an SLE in einer Nieren-Remission leidet.

12. Verfahren nach Anspruch 1, wobei die Probe eine Serum-Probe ist.

13. Verfahren nach Anspruch 1, wobei die Kontrolle ausgewählt ist aus der Gruppe bestehend aus einer Probe von mindestens einem gesunden Individuum, einem Panel Kontroll-Proben einer Gruppe gesunder Individuen, und einem gespeicherten Datensatz von Kontroll-Individuen.

14. Verfahren nach Anspruch 1, welches weiter Verdünnen der Probe 1:10 vor Bestimmen der Reaktivität von Antikörpern in der Probe umfasst.

15. Verfahren nach Anspruch 1, wobei die mehreren Antigene in Form eines Antigenarrays verwendet werden.

## Revendications

1. Procédé de diagnostic du lupus érythémateux disséminé (LED) chez un sujet, le procédé comprenant :
(i) la détermination de la réactivité d'anticorps IgG et IgM, dans un échantillon obtenu auprès du sujet, à au moins quatre antigènes choisis dans le groupe constitué de : IGFBP1, CD99, acide hyaluronique, EBV, ADNsb, ADNdb, MPO, cardiolipine, collagène III, collagène IV, actine, BMP4, CMV, F50, HGF, HSP60p18, RV, S100A4, CITED1 et FHIT, ce qui permet de déterminer le profil de réactivité de l'échantillon à la pluralité d'antigènes ; et
(ii) la comparaison du profil de réactivité dudit échantillon à un profil de réactivité témoin ;
dans lequel une réactivité accrue des anticorps IgG à une pluralité d'antigènes choisis dans le groupe constitué de : acide hyaluronique, EBV, ADNsb, ADNdb, BMP4, F50, HGF et HSP60p18, et/ou une réactivité diminuée des anticorps IgM à une pluralité d'antigènes choisis dans le groupe constitué de : IGFBP1, CD99, MPO, cardiolipine, collagène III, collagène IV, actine, CMV, RV, S100A4, CITED1 et FHIT par comparaison avec le profil de réactivité d'un échantillon témoin est une indication que le sujet souffre de LED.

2. Procédé selon la revendication 1, dans lequel la pluralité d'antigènes comprend au moins cinq antigènes.

3. Procédé selon la revendication 1, dans lequel la pluralité d'antigènes comprend au moins six antigènes.

4. Procédé selon la revendication 1, dans lequel la pluralité d'antigènes comprend au moins sept antigènes.

5. Procédé selon la revendication 1, dans lequel la pluralité d'antigènes comprend au moins huit antigènes.

6. Procédé selon la revendication 1, dans lequel la pluralité d'antigènes est constituée de IGFBP1, CD99, acide hyaluronique, EBV, ADNsb, ADNdb, MPO, cardiolipine et collagène III.

7. Procédé selon la revendication 1, comprenant la détermination de la réactivité d'une pluralité d'anticorps IgG et d'une pluralité d'anticorps IgM dans ledit échantillon à ladite pluralité d'antigènes.

8. Procédé selon la revendication 1, comprenant la détermination de la réactivité d'anticorps IgG dans l'échantillon obtenu auprès du sujet à une pluralité d'antigènes choisis dans le groupe constitué de : acide hyaluronique, EBV, ADNsb et ADNdb.

9. Procédé selon la revendication 1, comprenant la détermination de la réactivité d'anticorps IgM dans l'échantillon obtenu auprès du sujet à une pluralité d'antigènes choisis dans le groupe constitué de : CD99, IGFBP1, MPO, cardiolipine et collagène III.

10. Procédé selon la revendication 1, comprenant la détermination de la réactivité d'anticorps IgM dans l'échantillon obtenu auprès du sujet à une pluralité d'antigènes choisis dans le groupe constitué de : CD99, IGFBP1, MPO et cardiolipine.

11. Procédé selon la revendication 1, comprenant la détermination de la réactivité d'anticorps IgM dans l'échantillon obtenu auprès du sujet à une pluralité d'antigènes choisis dans le groupe constitué de : CD99, MPO et collagène III, dans lequel une régulation à la baisse entre le profil de réactivité dudit échantillon obtenu auprès du sujet par comparaison avec le profil de réactivité d'un échantillon témoin est une indication que le sujet souffre de LED en rémission rénale.

12. Procédé selon la revendication 1, dans lequel l'échantillon est un échantillon de sérum.

13. Procédé selon la revendication 1, dans lequel le témoin est choisi dans le groupe constitué d'un échantillon provenant d'au moins un sujet en bonne santé, un panel d'échantillons témoins provenant d'un ensemble de sujets en bonne santé, et un ensemble enregistré de données provenant de sujets témoins.

14. Procédé selon la revendication 1, comprenant en outre la dilution de l'échantillon 1:10 avant détermination de la réactivité d'anticorps dans l'échantillon.

15. Procédé selon la revendication 1, dans lequel ladite pluralité d'antigènes est utilisée sous la forme d'un jeu d'antigènes.


### SEQUENCE LISTING

<110> YEDA RESEARCH AND DEVELOPMENT CO. LTD.
<120> DIAGNOSIS OF SYSTEMIC LUPUS ERYTHEMATOSUS
<130> YEDA/0111 PCT
<150> US Prov 61/303,691
   <151> 2010-02-12
<160> 13
<170> PatentIn version 3.5
<210> 1
   <211> 85
   <212> PRT
   <213> Artifical
<400> 1
   Thr Ala Ser Glu Pro Glu Asp Lys Ser Pro Arg Val Gln Pro Leu Gly
   1               5                   10                  15
   Thr Gly Leu Gln Gln Arg Pro Arg His Thr Val Ser Pro Ser Pro Ser
               20                  25                  30
   Pro Pro Pro Pro Pro Arg Thr Pro Thr Trp Glu Ser Pro Ala Arg Pro
           35                  40                  45
   Glu Thr Pro Ser Pro Ala Ile Pro Ser His Ser Ser Asn Thr Ala Leu
       50                  55                  60
   Glu Arg Pro Leu Ala Val Gln Leu Ala Arg Lys Arg Thr Ser Ser Glu
   65                  70                  75                  80
   Ala Arg Gln Lys Gln
                   85
<210> 2
   <211> 185
   <212> PRT
   <213> Homo sapiens
<400> 2
   Met Ala Arg Gly Ala Ala Leu Ala Leu Leu Leu Phe Gly Leu Leu Gly
   1               5                   10                  15
   Val Leu Val Ala Ala Pro Asp Gly Gly Phe Asp Leu Ser Asp Ala Leu
               20                  25                  30
   Pro Asp Asn Glu Asn Lys Lys Pro Thr Ala Ile Pro Lys Lys Pro Ser
           35                  40                  45
   Ala Gly Asp Asp Phe Asp Leu Gly Asp Ala Val Val Asp Gly Glu Asn
       50                  55                  60
   Asp Asp Pro Arg Pro Pro Asn Pro Pro Lys Pro Met Pro Asn Pro Asn
   65                  70                  75                  80
   Pro Asn His Pro Ser Ser Ser Gly Ser Phe Ser Asp Ala Asp Leu Ala
                   85                  90                  95
   Asp Gly Val Ser Gly Gly Glu Gly Lys Gly Gly Ser Asp Gly Gly Gly
               100                 105                 110
   Ser His Arg Lys Glu Gly Glu Glu Ala Asp Ala Pro Gly Val Ile Pro
           115                 120                 125
   Gly Ile Val Gly Ala Val Val Val Ala Val Ala Gly Ala Ile Ser Ser
       130                 135                 140
   Phe Ile Ala Tyr Gln Lys Lys Lys Leu Cys Phe Lys Glu Asn Ala Glu
   145                 150                 155                 160
   Gln Gly Glu Val Asp Met Glu Ser His Arg Asn Ala Asn Ala Glu Pro
                   165                 170                 175
   Ala Val Gln Arg Thr Leu Leu Glu Lys
               180                 185
<210> 3
   <211> 745
   <212> PRT
   <213>> Homo sapiens
<400> 3
<210> 4
   <211> 259
   <212> PRT
   <213> Homo sapiens
<400> 4
   Met Ser Glu Val Pro Val Ala Arg Val Trp Leu Val Leu Leu Leu Leu
   1 5 10 15
   Thr Val Gln Val Gly Val Thr Ala Gly Ala Pro Trp Gln Cys Ala Pro
   20 25 30
   Cys Ser Ala Glu Lys Leu Ala Leu Cys Pro Pro Val Ser Ala Ser Cys
   35 40 45
   Ser Glu Val Thr Arg Ser Ala Gly Cys Gly Cys Cys Pro Met Cys Ala
   50 55 60
   Leu Pro Leu Gly Ala Ala Cys Gly Val Ala Thr Ala Arg Cys Ala Arg
   65 70 75 80
   Gly Leu Ser Cys Arg Ala Leu Pro Gly Glu Gln Gln Pro Leu His Ala
   85 90 95
   Leu Thr Arg Gly Gln Gly Ala Cys Val Gln Glu Ser Asp Ala Ser Ala
   100 105 110
   Pro His Ala Ala Glu Ala Gly Ser Pro Glu Ser Pro Glu Ser Thr Glu
   115 120 125
   Ile Thr Glu Glu Glu Leu Leu Asp Asn Phe His Leu Met Ala Pro Ser
   130 135 140
   Glu Glu Asp His Ser Ile Leu Trp Asp Ala Ile Ser Thr Tyr Asp Gly
   145 150 155 160
   Ser Lys Ala Leu His Val Thr Asn Ile Lys Lys Trp Lys Glu Pro Cys
   165 170 175
   Arg Ile Glu Leu Tyr Arg Val Val Glu Ser Leu Ala Lys Ala Gln Glu
   180 185 190
   Thr Ser Gly Glu Glu Ile Ser Lys Phe Tyr Leu Pro Asn Cys Asn Lys
   195 200 205
   Asn Gly Phe Tyr His Ser Arg Gln Cys Glu Thr Ser Met Asp Gly Glu
   210 215 220
   Ala Gly Leu Cys Trp Cys Val Tyr Pro Trp Asn Gly Lys Arg Ile Pro
   225 230 235 240
   Gly Ser Pro Glu Ile Arg Gly Asp Pro Asn Cys Gln Ile Tyr Phe Asn
   245 250 255
   Val Gln Asn
<210> 5
   <211> 116
   <212> PRT
   <213> Artificial
<220>
   <223> Synthetic peptide
<400> 5
   Ser Pro Lys His His Ser Gln Arg Ala Arg Lys Lys Asn Lys Asn Cys
   1 5 10 15
   Arg Arg His Ser Leu Tyr Val Asp Phe Ser Asp Val Gly Trp Asn Asp
   20 25 30
   Trp Ile Val Ala Pro Pro Gly Tyr Gln Ala Phe Tyr Cys His Gly Asp
   35 40 45
   Cys Pro Phe Pro Leu Ala Asp His Leu Asn Ser Thr Asn His Ala Ile
   50 55 60
   Val Gln Thr Leu Val Asn Ser Val Asn Ser Ser Ile Pro Lys Ala Cys
   65 70 75 80
   Cys Val Pro Thr Glu Leu Ser Ala Ile Ser Met Leu Tyr Leu Asp Glu
   85 90 95
   Tyr Asp Lys Val Val Leu Lys Asn Tyr Gln Glu Met Val Val Glu Gly
   100 105 110
   Cys Gly Cys Arg
   115
<210> 6
   <211> 191
   <212> PRT
   <213> Artificial
<220>
   <223> Synthetic peptide
<400> 6
   Thr Lys Ala Ser Pro Gly Arg Val Arg Arg Asp Ser Ala Trp Asp Val
   1 5 10 15
   Arg Pro Leu Thr Glu Thr Arg Gly Asp Leu Phe Ser Gly Asp Glu Asp
   20 25 30
   Ser Asp Ser Ser Asp Gly Tyr Pro Pro Asn Arg Gln Asp Pro Arg Phe
   35 40 45
   Thr Asp Thr Leu Val Asp Ile Thr Asp Thr Glu Thr Ser Ala Lys Pro
   50 55 60
   Pro Val Thr Thr Ala Tyr Lys Phe Gly Gln Pro Thr Leu Thr Phe Gly
   65 70 75 80
   Ala Gly Val Asn Val Pro Ala Gly Ala Gly Ala Ala Ile Leu Thr Pro
   85 90 95
   Thr Pro Val Asn Pro Ser Thr Ala Pro Ala Pro Ala Pro Thr Pro Thr
   100 105 110
   Phe Ala Gly Thr Gln Thr Pro Val Asn Gly Asn Ser Pro Trp Ala Pro
   115 120 125
   Thr Ala Pro Leu Pro Gly Asp Met Asn Pro Ala Asn Trp Pro Arg Glu
   130 135 140
   Arg Ala Trp Ala Leu Lys Asn Pro His Leu Ala Tyr Asn Pro Phe Arg
   145 150 155 160
   Met Pro Thr Thr Ser Thr Ala Ser Gln Asn Thr Val Ser Thr Thr Pro
   165 170 175
   Arg Arg Pro Ser Thr Pro Arg Ala Ala Val Thr Gln Thr Ala Ser
   180 185 190
<210> 7
   <211> 20
   <212> PRT
   <213> Artificial
<220>
   <223> Synthetic peptide
<400> 7
   Cys Val Lys Gly Gly Thr Thr Lys Ile Phe Leu Val Gly Asp Tyr Ser
   1 5 10 15
   Ser Ser Ala Glu
   20
<210> 8
   <211> 723
   <212> PRT
   <213> Homo sapiens
<400> 8
   Met Trp Val Thr Lys Leu Leu Pro Ala Leu Leu Leu Gln His Val Leu
   1 5 10 15
   Leu His Leu Leu Leu Leu Pro Ile Ala Ile Pro Tyr Ala Glu Gly Gln
   20 25 30
   Arg Lys Arg Arg Asn Thr Ile His Glu Phe Lys Lys Ser Ala Lys Thr
   35 40 45
   Thr Leu Ile Lys Ile Asp Pro Ala Leu Lys Ile Lys Thr Lys Lys Val
   50 55 60
   Asn Thr Ala Asp Gln Cys Ala Asn Arg Cys Thr Arg Asn Lys Gly Leu
   65                  70                  75                  80
   Pro Phe Thr Cys Lys Ala Phe Val Phe Asp Lys Ala Arg Lys Gln Cys
                   85                  90                  95
   Leu Trp Phe Pro Phe Asn Ser Met Ser Ser Gly Val Lys Lys Glu Phe
               100                 105                 110
   Gly His Glu Phe Asp Leu Tyr Glu Asn Lys Asp Tyr Ile Arg Asn Cys
           115                 120                 125
   Ile Ile Gly Lys Gly Arg Ser Tyr Lys Gly Thr Val Ser Ile Thr Lys
       130                 135                 140
   Ser Gly Ile Lys Cys Gln Pro Trp Ser Ser Met Ile Pro His Glu His
   145                 150                 155                 160
   Ser Tyr Arg Gly Lys Asp Leu Gln Glu Asn Tyr Cys Arg Asn Pro Arg
                   165                 170                 175
   Gly Glu Glu Gly Gly Pro Trp Cys Phe Thr Ser Asn Pro Glu Val Arg
               180                 185                 190
   Tyr Glu Val Cys Asp Ile Pro Gln Cys Ser Glu Val Glu Cys Met Thr
           195                 200                 205
   Cys Asn Gly Glu Ser Tyr Arg Gly Leu Met Asp His Thr Glu Ser Gly
       210                 215                 220
   Lys Ile Cys Gln Arg Trp Asp His Gln Thr Pro His Arg His Lys Phe
   225                 230                 235                 240
   Leu Pro Glu Arg Tyr Pro Asp Lys Gly Phe Asp Asp Asn Tyr Cys Arg
                   245                 250                 255
   Asn Pro Asp Gly Gln Pro Arg Pro Trp Cys Tyr Thr Leu Asp Pro His
               260                 265                 270
   Thr Arg Trp Glu Tyr Cys Ala Ile Lys Thr Cys Ala Asp Asn Thr Met
           275                 280                 285
   Asn Asp Thr Asp Val Pro Leu Glu Thr Thr Glu Cys Ile Gln Gly Gln
       290                 295                 300
   Gly Glu Gly Tyr Arg Gly Thr Val Asn Thr Ile Trp Asn Gly Ile Pro
   305                 310                 315                 320
   Cys Gln Arg Trp Asp Ser Gln Tyr Pro His Glu His Asp Met Thr Pro
                   325                 330                 335
   Glu Asn Phe Lys Cys Lys Asp Leu Arg Glu Asn Tyr Cys Arg Asn Pro
               340                 345                 350
   Asp Gly Ser Glu Ser Pro Trp Cys Phe Thr Thr Asp Pro Asn Ile Arg
           355                 360                 365
   Val Gly Tyr Cys Ser Gln Ile Pro Asn Cys Asp Met Ser His Gly Gln
       370                 375                 380
   Asp Cys Tyr Arg Gly Asn Gly Lys Asn Tyr Met Gly Asn Leu Ser Gln
   385                 390                 395                 400
   Thr Arg Ser Gly Leu Thr Cys Ser Met Trp Asp Lys Asn Met Glu Asp
                   405                 410                 415
   Leu His Arg His Ile Phe Trp Glu Pro Asp Ala Ser Lys Leu Asn Glu
               420                 425                 430
   Asn Tyr Cys Arg Asn Pro Asp Asp Asp Ala His Gly Pro Trp Cys Tyr
           435                 440                 445
   Thr Gly Asn Pro Leu Ile Pro Trp Asp Tyr Cys Pro Ile Ser Arg Cys
       450                 455                 460
   Glu Gly Asp Thr Thr Pro Thr Ile Val Asn Leu Asp His Pro Val Ile
   465                 470                 475                 480
   Ser Cys Ala Lys Thr Lys Gln Leu Arg Val Val Asn Gly Ile Pro Thr
                   485                 490                 495
   Arg Thr Asn Ile Gly Trp Met Val Ser Leu Arg Tyr Arg Asn Lys His
               500                 505                 510
   Ile Cys Gly Gly Ser Leu Ile Lys Glu Ser Trp Val Leu Thr Ala Arg
           515                 520                 525
   Gln Cys Phe Pro Ser Arg Asp Leu Lys Asp Tyr Glu Ala Trp Leu Gly
       530                 535                 540
   Ile His Asp Val His Gly Arg Gly Asp Glu Lys Cys Lys Gln Val Leu
   545                 550                 555                 560
   Asn Val Ser Gln Leu Val Tyr Gly Pro Glu Gly Ser Asp Leu Val Leu
                   565                 570                 575
   Met Lys Leu Ala Arg Pro Ala Val Leu Asp Asp Phe Val Ser Thr Ile
               580                 585                 590
   Asp Leu Pro Asn Tyr Gly Cys Thr Ile Pro Glu Lys Thr Ser Cys Ser
           595                 600                 605
   Val Tyr Gly Trp Gly Tyr Thr Gly Leu Ile Asn Tyr Asp Gly Leu Leu
       610                 615                 620
   Arg Val Ala His Leu Tyr Ile Met Gly Asn Glu Lys Cys Ser Gln His
   625                 630                 635                 640
   His Arg Gly Lys Val Thr Leu Asn Glu Ser Glu Ile Cys Ala Gly Ala
                   645                 650                 655
   Glu Lys Ile Gly Ser Gly Pro Cys Glu Gly Asp Tyr Gly Gly Pro Leu
               660                 665                 670
   Val Cys Glu Gln His Lys Met Arg Met Val Leu Gly Val Ile Val Pro
           675                 680                 685
   Gly Arg Gly Cys Ala Ile Pro Asn Arg Pro Gly Ile Phe Val Arg Val
       690                 695                 700
   Ala Tyr Tyr Ala Lys Trp Ile His Lys Ile Ile Leu Thr Tyr Lys Val
   705                 710                 715                 720
   Pro Gln Ser
<210> 9
   <211> 21
   <212> PRT
   <213> Artificial
<220>
   <223> Synthetic peptide
<400> 9
   Gln Ser Ile Val Pro Ala Leu Glu Ile Ala Asn Ala His Arg Lys Pro
   1               5                   10                  15
   Leu Val Ile Ile Ala
               20
<210> 10
   <211> 123
   <212> PRT
   <213> Artificial
<220>
   <223> Synthetic peptide
<400> 10
   Met Ala Ser Thr Thr Pro Ile Thr Met Glu Asp Leu Gln Lys Ala Leu
   1 5 10 15
   Glu Ala Gln Ser Arg Ala Leu Arg Ala Glu Leu Ala Ala Gly Ala Ser
   20 25 30
   Gln Ser Arg Arg Pro Arg Pro Pro Arg Gln Arg Asp Ser Ser Thr Ser
   35 40 45
   Gly Asp Asp Ser Gly Arg Asp Ser Gly Gly Pro Arg Arg Arg Arg Gly
   50 55 60
   Asn Arg Gly Arg Gly Gln Arg Arg Asp Trp Ser Arg Ala Pro Pro Pro
   65 70 75 80
   Pro Glu Glu Arg Gln Glu Thr Arg Ser Gln Thr Pro Ala Pro Lys Pro
   85 90 95
   Ser Arg Ala Pro Pro Gln Gln Pro Gln Pro Pro Arg Met Gln Thr Gly
   100 105 110
   Arg Gly Gly Ser Ala Pro Arg Pro Glu Leu Gly
   115 120
<210> 11
   <211> 101
   <212> PRT
   <213> Homo sapiens
<400> 11
   Met Ala Cys Pro Leu Glu Lys Ala Leu Asp Val Met Val Ser Thr Phe
   1 5 10 15
   His Lys Tyr Ser Gly Lys Glu Gly Asp Lys Phe Lys Leu Asn Lys Ser
   20 25 30
   Glu Leu Lys Glu Leu Leu Thr Arg Glu Leu Pro Ser Phe Leu Gly Lys
   35 40 45
   Arg Thr Asp Glu Ala Ala Phe Gln Lys Leu Met Ser Asn Leu Asp Ser
   50 55 60
   Asn Arg Asp Asn Glu Val Asp Phe Gln Glu Tyr Cys Val Phe Leu Ser
   65 70 75 80
   Cys Ile Ala Met Met Cys Asn Glu Phe Phe Glu Gly Phe Pro Asp Lys
   85 90 95
   Gln Pro Arg Lys Lys
   100
<210> 12
   <211> 193
   <212> PRT
   <213> Homo sapiens
<400> 12
   Met Pro Thr Thr Ser Arg Pro Ala Leu Asp Val Lys Gly Gly Thr Ser
   1 5 10 15
   Pro Ala Lys Glu Asp Ala Asn Gln Glu Met Ser Ser Val Ala Tyr Ser
   20 25 30
   Asn Leu Ala Val Lys Asp Arg Lys Ala Val Ala Ile Leu His Tyr Pro
   35 40 45
   Gly Val Ala Ser Asn Gly Thr Lys Ala Ser Gly Ala Pro Thr Ser Ser
   50 55 60
   Ser Gly Ser Pro Ile Gly Ser Pro Thr Thr Thr Pro Pro Thr Lys Pro
   65 70 75 80
   Pro Ser Phe Asn Leu His Pro Ala Pro His Leu Leu Ala Ser Met His
   85 90 95
   Leu Gln Lys Leu Asn Ser Gln Tyr Gln Gly Met Ala Ala Ala Thr Pro
   100 105 110
   Gly Gln Pro Gly Glu Ala Gly Pro Leu Gln Asn Trp Asp Phe Gly Ala
   115 120 125
   Gln Ala Gly Gly Ala Glu Ser Leu Ser Pro Ser Ala Gly Ala Gln Ser
   130 135 140
   Pro Ala Ile Ile Asp Ser Asp Pro Val Asp Glu Glu Val Leu Met Ser
   145 150 155 160
   Leu Val Val Glu Leu Gly Leu Asp Arg Ala Asn Glu Leu Pro Glu Leu
   165 170 175
   Trp Leu Gly Gln Asn Glu Phe Asp Phe Thr Ala Asp Phe Pro Ser Ser
   180 185 190
   Cys
<210> 13
   <211> 48
   <212> PRT
   <213> Artificial
<220>
   <223> synthetic peptide
<400> 13
   Val Leu Val Cys Pro Leu Arg Pro Val Glu Arg Phe His Asp Leu Arg
   1               5                   10                  15
   Pro Asp Glu Val Ala Asp Leu Phe Gln Thr Thr Gln Arg Val Gly Thr
               20                  25                  30
   Val Val Glu Lys His Phe His Gly Thr Ser Leu Thr Phe Ser Met Gln
           35                  40                  45